# EUROPÄISCHE PATENTSCHRIFT

(11) **EP 0 680 471 B1**
(45) Veröffentlichungstag und Bekanntmachung des Hinweises auf die Patenterteilung: **10.06.1998**
(21) Anmeldenummer: 94905037.1
(22) Anmeldetag: 12.01.1994
(51) Int. Cl.: C07D 221/16, A01N 43/42

(54) **5-SUBSTITUIERTE 4-METHYL-5H-INDENO[1,2-b]PYRIDINE, DEREN HERSTELLUNG UND VERWENDUNG ALS HERBIZIDE UND PFLANZENWACHSTUMSREGULATOREN**
5-SUBSTITUTED 4-METHYL-5H-INDENO 1,2-b]PYRIDINES, THEIR PREPARATION AND USE AS HERBICIDES AND PLANT GROWTH REGULATORS
4-METHYL-5H-INDENO 1,2-b]PYRIDINES SUBSTITUEES EN POSITION 5, LEUR PREPARATION ET LEUR UTILISATION COMME HERBICIDES ET COMME REGULATEURS DE LA CROISSANCE VEGETALE

(30) Priorität: 20.01.1993 DE 4301426
(43) Veröffentlichungstag der Anmeldung: 08.11.1995
(73) Patentinhaber: BASF AKTIENGESELLSCHAFT, 67056 Ludwigshafen (DE)
(72) Erfinder: RENTZEA, Costin, D-69123 Heidelberg (DE); MEYER, Norbert, D-68526 Ladenburg (DE); KAST, Juergen, D-67459 Boehl-Iggelheim (DE); PLATH, Peter, D-67227 Frankenthal (DE); KOENIG, Hartmann, D-67117 Limburgerhof (DE); HARREUS, Albrecht, D-67063 Ludwigshafen (DE); KARDORFF, Uwe, D-68159 Mannheim (DE); GERBER, Matthias, D-67117 Limburgerhof (DE); WALTER, Helmut, D-67238 Obrigheim (DE); LANDES, Andreas, D-67117 Limburgerhof (DE); WESTPHALEN, Karl-Otto, D-67436 Speyer (DE)
(86) Internationale Anmeldenummer: EP9400071
(87) Internationale Veröffentlichungsnummer: WO9417044

(56) Entgegenhaltungen:
- EP-A- 0 369 426
- WO-A-93/21162
- CHEMICAL ABSTRACTS, Vol. 57, Nr. 2, ver!ffentlicht 1962, Juli 23, Columbus, Ohio, USA J. BEGER et al. "2-Azafluore- nes from 4-aryl-3-carboxy- pyridines"

## Beschreibung

Die vorliegende Erfindung betrifft 4-Methyl-5H-indeno[1,2-b]pyridine und 1-Methyl-9H-indeno[2,1-c]pyridine der allgemeinen Formel I 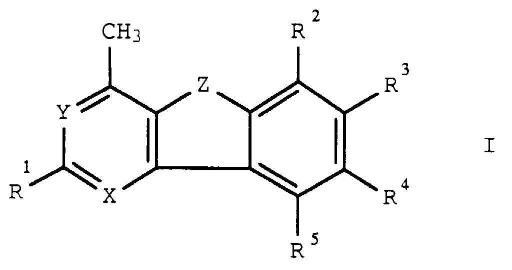 in der X, Y, Z und die Reste R¹ bis R⁵ folgende Bedeutung haben:
- R¹: Wasserstoff, C₁-C₆-Alkyl, eine Gruppe COR⁶, wobei R⁶ für C₁-C₄-Alkyl steht,
- R²-R⁵: a) Wasserstoff,
b) Halogen,
c) Nitro,
d) COOR⁶, wobei R⁶ für C₁-C₄-Alkyl steht,
e) CONH₂ und CONR⁶R⁷, wobei R⁶ und R⁷ C₁-C₄-Alkyl bedeuten,
f) C₁-C₈-Alkyl, das ein bis drei der folgenden Substituenten tragen kann: Halogen, Hydroxy, C₁-C₆-Alkoxy,
g) C₃-C₆-Alkenyl,
h) C₁-C₄-Alkoxy,
i) Hydroxy,
j) Amino oder NR⁶R⁷,
k) Phenyl, das ein bis fünf Halogenatome oder ein bis drei der folgenden Substituenten tragen kann: Nitro, C₁-C₄-Alkoxy, C₁-C₄-Alkyl oder C₁-C₄-Halogenalkyl;
- X: N,N⁺-O⁻;
- Y: CR⁸, wobei R⁸ für Wasserstoff, C₁-C₂-Alkyl, COOH oder COOR⁶ steht;
- Z: eine Gruppe C=O, CH-OR⁹ CH-O-COR⁹ oder C=N-W-R⁹, wobei W für Sauerstoff oder die Gruppierung -N(R¹⁰) steht und in der
- R⁹: Wasserstoff, eine C₁-C₆-Alkylgruppe, die durch Halogen, COOR⁶ oder C₁-C₄-Alkoxy substituiert sein kann, eine C₃-C₆-Alkenylgruppe, eine C₃-C₆-Halogenalkenylgruppe, eine C₃-C₆-Alkinylgruppe, ferner Phenyl-, Phenyl-C₁-C₄-alkyl oder einkernige Heteroaryl- oder Heteroaryl-C₁-C₄-alkylreste bedeutet, wobei Heteroaryl jeweils für einen heteroaromatischen Rest mit 5 bis 6 Ringgliedern, enthaltend 1 bis 3 Heteroatome wie N, S oder O steht und wobei diese aromatischen bzw. heteroaromatischen Gruppen ein bis drei der folgenden Substituenten am Kern tragen können: Halogen, Nitro, C₁-C₆-Alkyl, C₁-C₆-Alkoxy, C₁-C₂-Halogenalkyl, Amino, C₁-C₆-Alkylamino, Di(C₁-C₆-alkyl)amino und
- R¹⁰: Wasserstoff oder eine C₁-C₄-Alkylgruppe bedeutet,
sowie die Salze von I mit solchen Säuren, welche die herbizide bzw. die pflanzenwachstumsregulierende Wirkung von I nicht beeinträchtigen, ausgenommen Onychin, 6-, 7-, 8- und 9-Methoxyonychin, 6-, 7-, 8- und 9-Hydroxyonychin, 7,8-Dimethoxyonychin und 4-Methyl-5H-indeno[1,2-b]pyridine der Formel Ia 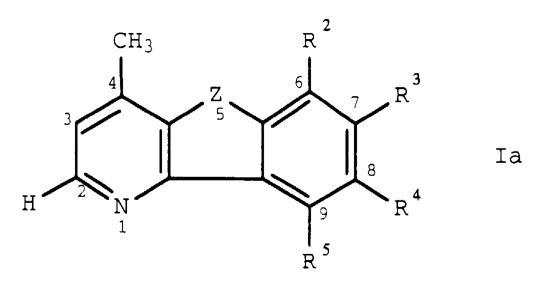 in der zwei der Reste R², R³, R⁴ oder R⁵ eine Kombination aus Methoxy und Hydroxy bilden, wenn Z für C=O oder CHOH steht und die verbleibenden zwei Reste Wasserstoff oder Methoxy bedeuten.

Aus den Arbeiten von J. Koyama et al (Heterocycles 12, 1017-1019 (1979)) und von V. Snieckus et al (Tetrahedron Lett., 29, 2135-2136 (1988)) ist das Alkaloid Onychin oder 4-Methyl-5H-indeno[1,2-b]pyridin-5-on bekannt, das aus der Pflanze Onychopetalum amazonicum isoliert wurde. Bereits bekannt sind auch weitere, mit Onychin strukturell verwandte Alkaloide, wie zum Beispiel das 8-Methoxy-onychin (M. O. F. Goulart et al. Phytochemistry 25, 1691-95 (1986) und Phytochemistry 26, 1551-1552 (1987)) isoliert aus Guatteria dielsiana, das Kinabalin oder 6,9-Dimethoxy-8-hydroxyonychin (D. Tadic et al, Phytochemistry 26, 537-541 (1987)) aus Meiogyne virgata, Darienin oder 8,9-Dimethoxy-7-hydroxyonychin und Macondin oder 6-Methoxy-7-hydroxyonychin (A. Cave et al, Phytochemistry 26, 2093-2098 (1987)) aus Oxandra xylopioides, das Ursulin oder 8-Hydroxy-9-methoxyonychin und Isoursulin bzw. Oxylopin oder 9-Hydroxy-8-methoxyonychin (A. Cave et al, Heterocycles 27, 407-421 (1988)) aus Unonopsis spectabilis, das Polylongin oder 4-Methyl-9-hydroxy-8-methoxy-5H-indeno[1,2-b]pyridin-5-ol (Yang Chang Wu, Heterocycles 29, 463-475 (1989)) aus Polyalthia longifolia, das Oncodin oder 7-Methoxy-8-hydroxyonychin (A. Cave et al, J. Natural Prod., 52, 273-278 (1989)) aus Oncostigma monosperma und Polyfothin oder 7,8-Dimethoxyonychin und Isooncodin oder 7-Hydroxy-8-methoxyonychin (Yang Chang Wu et al, J. Natural Prod., 53, 1327-1331 (1990)) aus Polyalthia longifolia.

Diesen Alkaloiden werden in der genannten Literatur antitumor, antivirale, anticandida und fungizide Eigenschaften zugeschrieben. Eine herbizide bzw. pflanzenwachstumsregulierende Wirkung dieser Naturstoffe ist jedoch nicht bekannt.

Es ist weiterhin bekannt, daß bestimmte Indeno-pyridazine (JO 3161478-A) und Indeno-pyrazole (GB-A-2 223 946) herbizide Eigenschaften besitzen.

Da die bekannten Naturstoffe in ihrer Wirkung nicht immer befriedigen, lag der Erfindung die Aufgabe zugrunde, neue herbizide Mittel mit stärkerem herbiziden Effekt und neue Pflanzenwachstumsregulatoren, vorzugsweise Wachstumshemmer zur Verfügung zu stellen. Weiterhin lagen der Erfindung neue herbizid und pflanzenwachstumsregulierend wirkende Verbindungen als Aufgabe zugrunde.

Demgemäß wurden die eingangs definierten neuen Verbindungen I gefunden.

Weiterhin betrifft die Erfindung herbizide und bioregulatorisch wirkende, insbesondere das Wachstum der Pflanzen hemmende Mittel, enthaltend die Verbindungen I und Verfahren zur Herstellung der Verbindungen I. Darüber hinaus wurde gefunden, daß die Verbindungen der Formel I', die den Verbindungen der Formel I, einschließlich den Verbindungen Ia entsprechen, zur Verwendung als Herbizide bzw. Pflanzenwachstumsregulatoren besonders geeignet sind.

Bevorzugte Verbindungen der Formel I sind solche, in denen die Substituenten folgende Bedeutung haben:
R¹
   Wasserstoff, C₁-C₆-Alkyl, insbesondere C₁-C₄-Alkyl, z.B. Methyl, Ethyl, Propyl sowie COR⁶ wie Acetyl;
R², R³, R⁴, R⁵
   Wasserstoff, Fluor, Chlor, Brom, unverzweigte oder verzweigte C₁-C₈-Alkyl, insbesondere C₁-C₆-Alkyl wie Methyl, Ethyl, n-Propyl, iso-Propyl, n-Butyl, sek.-Butyl, iso-Butyl, tert.Butyl, n-Pentyl, iso-Pentyl, 1,1-Dimethylpropyl, 2,2-Dimethylpropyl, n-Hexyl, 1-Ethyl-1-methylpropyl,
   eine unverzweigte oder verzweigte C₁-C₈-Alkylgruppe, insbesondere eine C₁-C₆-Alkylgruppe, die bis zu 3 der folgenden Substituenten tragen kann: Halogen wie Fluor, Chlor oder Brom, Hydroxyl, C₁-C₆-Alkoxy wie Methoxy, Ethoxy und Propoxy;
   eine unverzweigte oder verzweigte C₃-C₆-Alkenylgruppe, insbesondere Vinyl, Allyl, 2-Methylallyl, 3-Methylallyl, 2,3-Dimethylallyl, 3,3-Dimethylallyl, 2-Pentenyl und 3-Pentenyl;
   eine C₁-C₄-Alkoxygruppe, insbesondere Methoxy, Ethoxy und Propoxy,
   Nitro, Amino (NH₂), Estergruppierungen wie z. B. COOCH₃, COOC₂H₅, COOC₃H₇; Amide wie CO-NH₂ oder CO-N(CH₃)₂ und CO-N(C₂H₅)₂;
   eine Phenylgruppe, ggf. ein bis dreimal durch Fluor, Chlor, CF₃, NO₂ oder C₁-C₄-Alkyl substituiert;
R⁶, R⁷
   eine unverzweigte oder verzweigte C₁-C₄-Alkylgruppe wie Methyl, Ethyl, n-Propyl, iso-Propyl, n-Butyl, iso-Butyl, sek.-Butyl und tert.-Butyl;
R⁸
   Wasserstoff, Methyl, Ethyl, COOH, COOCH₃, COOC₂H₅ und COOC₃H₇;
R⁹
   Wasserstoff, C₁-C₆-Alkyl wie Methyl, Ethyl, n-Propyl, isoPropyl, n-Butyl, iso-Butyl, sek.-Butyl, tert.-Butyl, n-Pentyl, iso-Pentyl und n-Hexyl, wobei die Alkylgruppe durch Halogen wie Fluor, Chlor, Brom oder Iod, Methoxy, Ethoxy oder COOCH₃, COOC₂H₅ substituiert sein kann;
   C₃-C₆-Alkenyl wie Ethenyl, 2-Propenyl, 2-Butenyl, 2-Methyl-2propenyl, 3-Butenyl, 2-Pentenyl, 3-Methyl-2-butenyl und 3-Pentenyl;
   C₃-C₆-Halogenalkenyl wie 2-Chlor-2-propenyl, 3-Chlor-2-propenyl, 2-Brom-2-propenyl, 3-Chlor-2-butenyl, 2,3,3-Trichlor-2-propenyl;
   die Phenyl-, Phenyl-C₁-C₄-alkylgruppe oder ein einkerniger Heteroaryl-oder Heteroaryl-C₁-C₄-alkylrest, wobei Heteroaryl jeweils für einen heteroaromatischen Rest mit 5 bis 6 Ringgliedern, enthaltend 1 bis 3 Heteroatome wie N, S oder O steht. Beispielsweise seien 2-Pyrrolyl, 2-Thienyl, 3-Thienyl, 3-Furanyl, 4-Pyridyl, 3-Pyridyl, 2-Pyridyl, 2-Thiazolyl, 3-, 4- oder 5-Isoxazolyl genannt.

Die vorstehend genannten aromatischen und heteroaromatischen Gruppen können einen bis drei der folgenden Substituenten enthalten: Halogen wie Fluor, Chlor und Brom, Nitro, C₁-C₆-Alkyl wie Methyl, Ethyl, n-Propyl, iso-Propyl, n-Butyl, iso-Butyl, sek.-Butyl, tert.-Butyl, n-Pentyl, iso-Pentyl, C₁-C₆-Alkoxy wie Methoxy, Ethoxy, Propoxy, n-Butoxy, iso-Butoxy und tert.-Butoxy, C₁-C₂-Halogenalkyl wie Trifluormethyl oder Trichlormethyl und Amino, C₁-C₆-Alkylamino wie Methylamino und Ethylamino oder Di-(C₁-C₆)alkylamino wie Dimethylamino;
- R¹⁰: Wasserstoff, Methyl, Ethyl oder Propyl;
- X: N, N⁺-O⁻;
- Y: CH;
- Z: eine Gruppe C=O, CH-OR⁹, CH-O-COR⁹ oder C=N-W-R⁹,
- W: Sauerstoff oder die Gruppierung -N(R¹⁰).

Bevorzugte neue Indenopyridine der Formel I sind in den Tabellen 1 und 2 aufgeführt.

Als Säureadditionssalze eignen sich die Salze von solchen Säuren, welche die herbizide Wirkung der Verbindungen I und Ia nicht beeinträchtigen, also z. B. die Hydrochloride und -bromide, Sulfate, Nitrate, Phosphate, Oxalate oder die Dodecylbenzolsulfonte.

Die 4-Methyl-5H-indeno[1,2-b]pyridine Ia sind bekannt oder (im Falle I) in an sich bekannter Weise erhältlich.

In den Fällen in denen Y=CH, X=N, R¹=H und Z die Gruppe C=O bedeutet, kann man die Verbindungen I bzw. I' nach folgendem Schema herstellen:

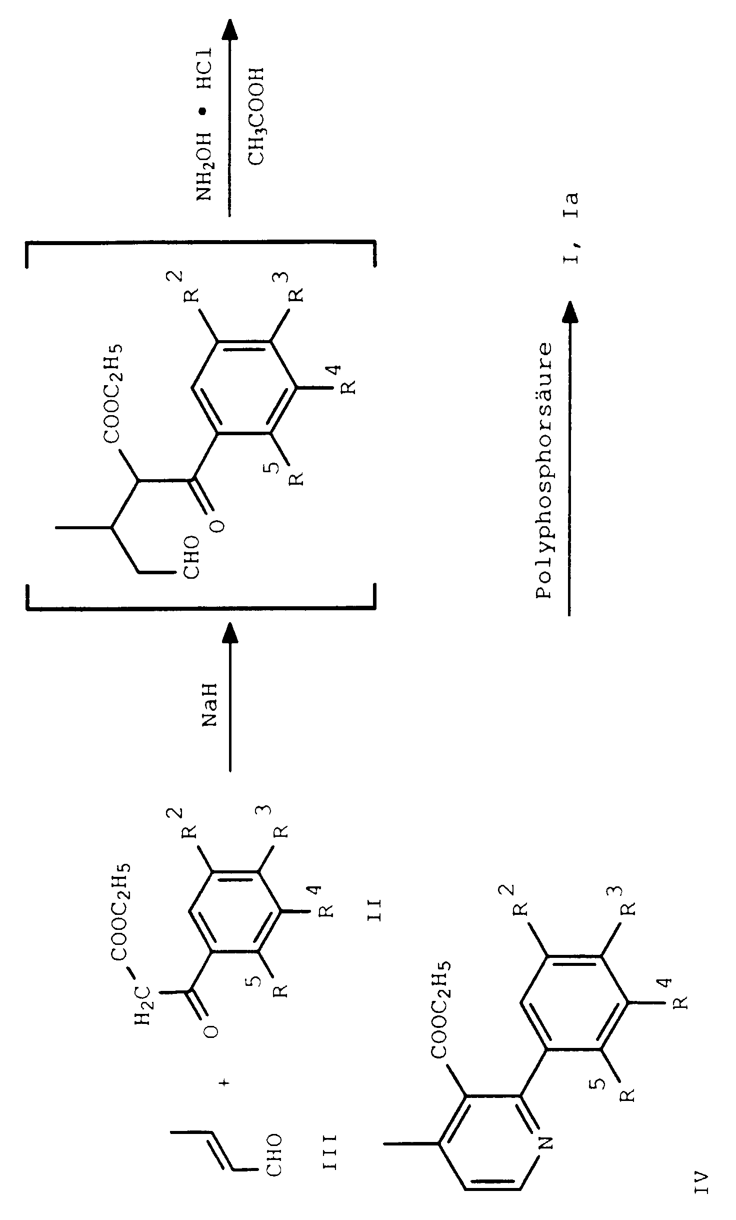

Dieses Verfahren ist z. B. aus den Arbeiten von F. Bracher (Liebigs Ann. Chem., 1988, 87 und Arch. Pharm., 322, 293 (1989)) bekannt.

Dabei wird die Michael-Addition der Benzoylessigester II an Crotonaldehyd III in einem inerten Lösungsmittel, z.B. in Dioxan mit einem Alkalimetallhydrid, z.B. Natriumhydrid als Katalysator und die weitere Cyclisierung zum 4-Methyl-2-aryl-nicotinsäureester IV, vorzugsweise mit Hydroxylamin-chlorhydrat in einer organischen Säure, z.B. Essigsäure durchgeführt. Die anschließende Cyclisierung zu den Verbindungen I bzw. Ia mit Polyphosphorsäure findet zwischen 90 und 180°C, bevorzugt zwischen 100 und 170°C statt, wobei man die Polyphosphorsäure vorteilhaft auch als Lösungs- und Verdünnungsmittel für die Reaktion verwenden kann. Die für die Synthese notwendigen Ausgangsstoffe sind entweder käuflich oder werden nach allgemein bekannten Verfahren hergestellt. Sie werden vorzugsweise in etwa stöchiometrischen Molverhältnissen eingesetzt.

In den Fällen in denen Y=CH, X=N, R¹≠H und Z die Gruppe C=O bedeutet, werden die Verbindungen I bzw. Ia bevorzugt nach dem einstufigen Verfahren von E. Breitmaier und E. Bayer (Angew. Chem. Int. Ed. Engl., 8, 865 (1969)) synthetisiert:

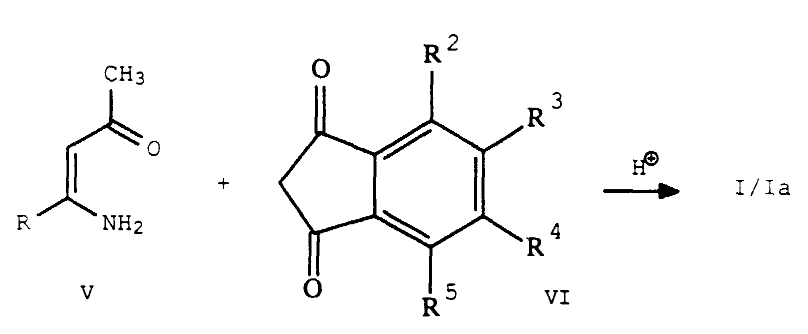

Vorzugsweise führt man diese Cyclisierung in einem aciden Medium, z.B. in organischen Säuren wie z. B. Ameisensäure, Essigsäure, Propionsäure oder Trifluoressigsäure, die bis zu 30 % Wasser enthalten dürfen, durch. Es kann auch vorteilhaft sein, die Umsetzung in Gegenwart von Ammoniumformiat oder Ammoniumacetat bzw. - propionat (bis zu stöchiometrische Mengen) vorzunehmen. Die Reaktionstemperaturen betragen im allgemeinen 70 bis 140°C, vorzugsweise 80 bis 130°C.

Die Enaminone V können z. B. nach P. C. Baraldi et al (Synthesis, 1983, 902) und die Dihydroindan-1,3-Dione VI z. B. nach A. R. Murthy et al (J. Med. Chem., 28, 1591 (1985)) hergestellt werden.

1-Methyl-9H-indeno[2,1-c]pyridine der Formel I mit R¹≠H, X=CH oder COOH, Y=N und Z= die Gruppe C=O können durch Cyclisierung von 4-Arylnicotinsäureestern oder von 4-Arylpyridin-3,5-dicarbonsäureestern in Polyphosphorsäure bei Temperaturen von 100 bis 180°C erhalten werden:

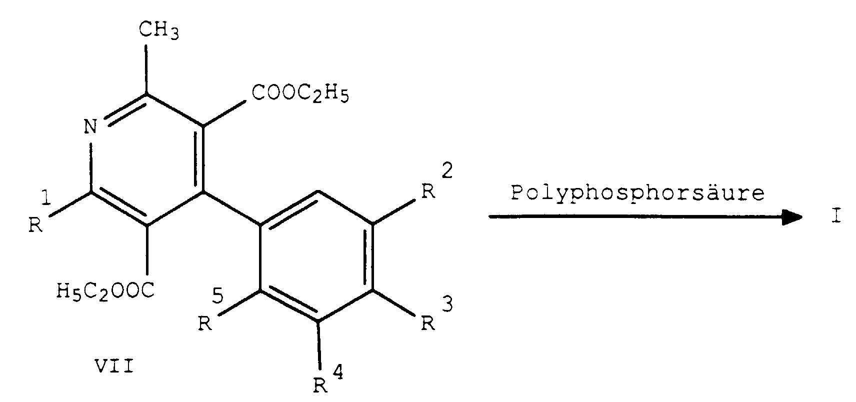

Die 4-Aryl-3,5-dicarbonsäureester VII können durch Natriumnitritoxidation (vgl. K. Görlitzer et al, Arch. Pharm. 314, 949 (1981)) der entsprechenden 1,4-Dihydropyridin-Derivate erhalten werden, die wiederum durch Hantzsch-Synthese zugänglich sind (s. z. B. B. Loev et al, J. Med. Chem., 17, 956 (1974)).

Die Synthese der Verbindungen I mit X=N⁺-O⁻ erfolgt durch Umsetzung der entsprechenden Indenopyridine mit H₂O₂ oder mit organischen Peroxysäuren nach Standardverfahren (s. z. B. E. Ochiai, "Aromatic Amine Oxides", Elsevier, Amsterdam, 1967, S. 200 - 250).

Verbindungen der Formel I/Ia mit Z=CO, können zu den entsprechenden Alkoholen (Z=CH-OH) mit komplexen Hydriden, vorzugsweise mit Natriumborhydrid reduziert werden. Vorzugsweise führt man die Reaktion in einem protischen Verdünnungsmittel wie z. B. Methanol, Ethanol oder iso-Propanol bei Temperaturen von -10 bis 50°C, vorzugsweise 0 bis 30°C durch.

Die Ketone der Formel I/Ia können auch mit sekundären Alkoholaten (vorzugsweise 0,3 bis 1,5 Moläquivalente), vorzugsweise des Aluminiums, wie z. B. Aluminiumisopropylat, Aluminiumbutylat-(2) oder Aluminiumcyclohexylat in Gegenwart eines Verdünnungsmittels bei 60 bis 160°C, vorzugsweise bei der Siedetemperatur des Verdünnungsmittels umgesetzt werden. Als Verdünnungsmittel eignen sich inerte organische Lösungsmittel, insbesondere niedermolekulare Alkohole wie Isopropanol oder Cyclohexanol. Die entstandenen Alkoholate werden sodann mit Hilfe von Säuren in übliche Weise zu den freien Alkoholen der Formel I/Ia mit Z=CHOH hydrolysiert.

Das Verfahren zur Herstellung der Ester der Formel I/Ia (Z=CH-O-COR⁹) besteht darin, daß man die Alkohole I/Ia (Z=CH-OH) mit den entsprechenden Säurechloriden oder Säureanhydriden in Gegenwart eines säurebindenden Mittels und gegebenenfalls in Gegenwart eines aprotischen Lösungsmittels oder Verdünnungsmittels sowie bevorzugt in Gegenwart eines Acylierungskatalysators bei Temperaturen zwischen 0 und 100°C, vorzugsweise zwischen 10 und 50°C umsetzt. Als säurebindende Mittel können anorganische Basen wie Natriumcarbonat, Kaliumcarbonat, Natriumhydrid oder besonders bevorzugt Pyridin oder Triethylamin in mindestens äquivalenten Mengen eingesetzt werden. Als Acylierungskatalysatoren verwendet man zweckmäßigerweise Imidazol oder 4-Dimethylaminopyridin in Mengenanteilen von 0,01 bis 0,4 Äquivalenten, falls nicht bereits Pyridin anwesend ist. Als Lösungsmittel können Kohlenwasserstoffe wie Cyclohexan oder Toluol, Ether wie Diethylether oder Tetrahydrofuran oder auch überschüssige säurebindende Amine wie Triethylamin oder Pyridin eingesetzt werden.

Vorzugsweise führt man die Herstellung der Oxime und der Hydrazone I/Ia (Z=C=N-W-R⁹) in Alkoholen wie Methanol, Ethanol oder Iso-Propanol oder in organischen Säuren wie Essigsäure oder deren Gemische, bei Temperaturen von 50 - 130°C, vorzugsweise bei der Siedetemperatur des Lösungsmittels durch.

Bevorzugt werden stoechiometrische Mengen oder ein geringer Überschuß des O-substituierten Hydroxylamins oder des Hydrazins eingesetzt.

Im Hinblick auf die biologische Verwendung bevorzugte Indenopyridine der allgemeinen Formel I bzw. I' sind in der nachfolgenden Tabelle 1 und in Tabelle 2 aufgeführt. Besonders bevorzugt sind Verbindungen der Formel I, in der mindestens einer (insbesondere ein bis drei) der Reste R²-R⁵ für C₁-C₄-Halogenalkyl oder für Halogen, insbesondere Fluor, Chlor oder Brom steht. Bevorzugt sind ferner Verbindungen mit R², R³, R⁴ oder R⁵ = Alkyl, insbesondere verzweigtes Alkyl oder Cycloalkyl wie Cyclohexyl, Cyclopropyl oder Cyclopentyl. Daneben sind Verbindungen bevorzugt, worin 1, 2 oder 3 der Reste R²-R⁵ für NO₂, NH₂, CONH₂ oder Phenyl stehen. Sofern nicht alle Reste R²-R⁵ Wasserstoff bedeuten, sind vorzugsweise ein, zwei oder drei Substituenten ungleich Wasserstoff am Phenylring.

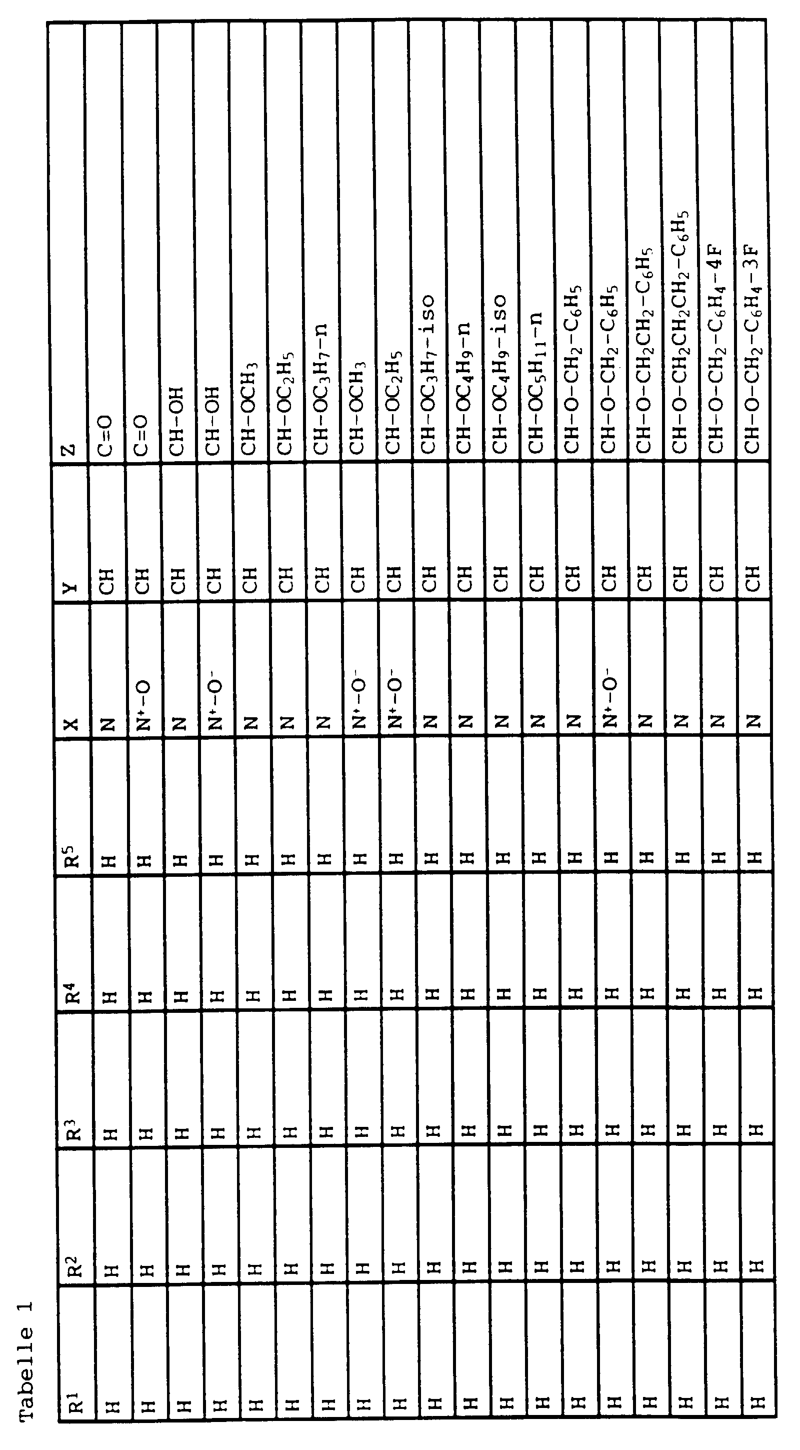

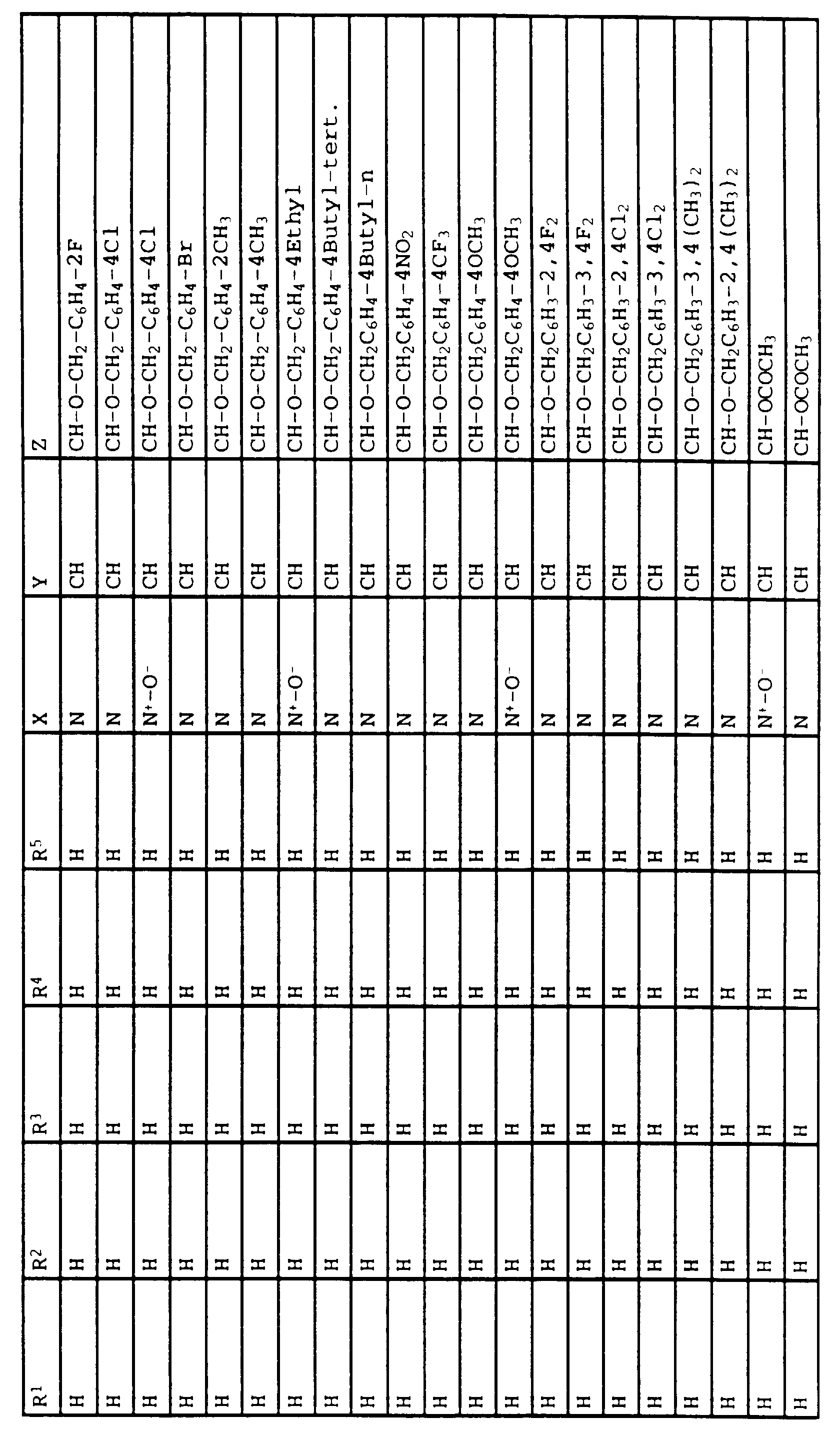

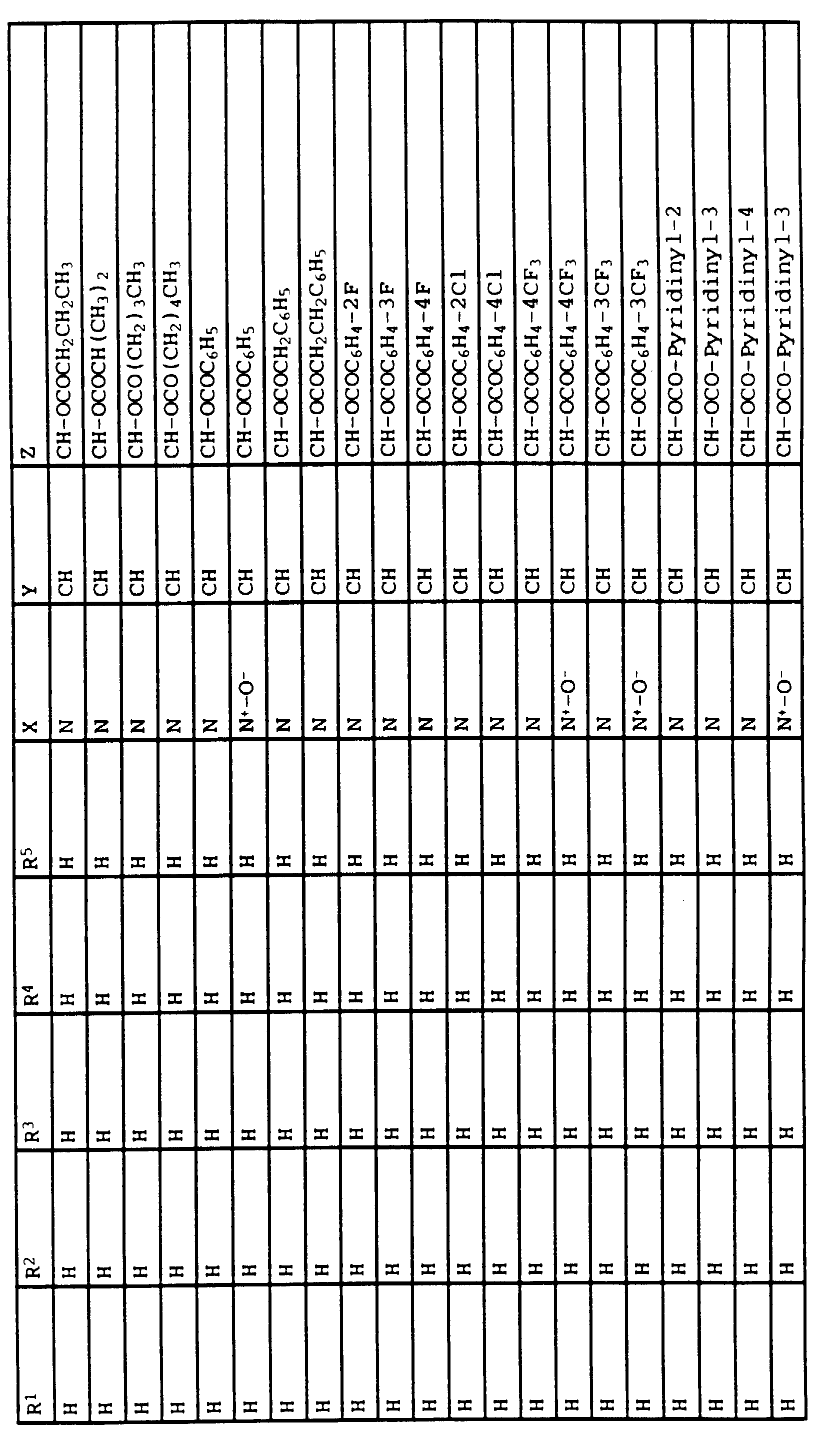

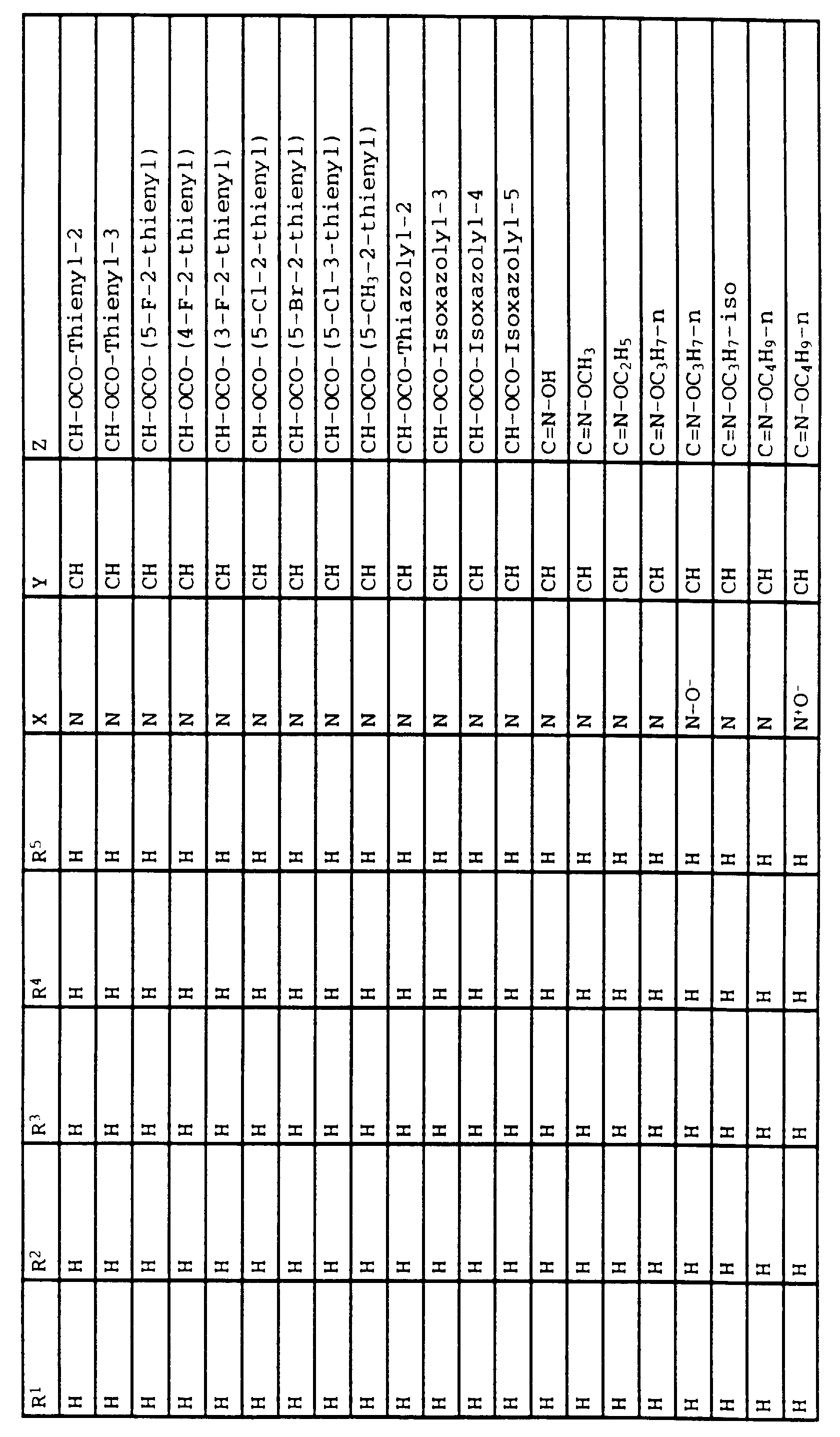

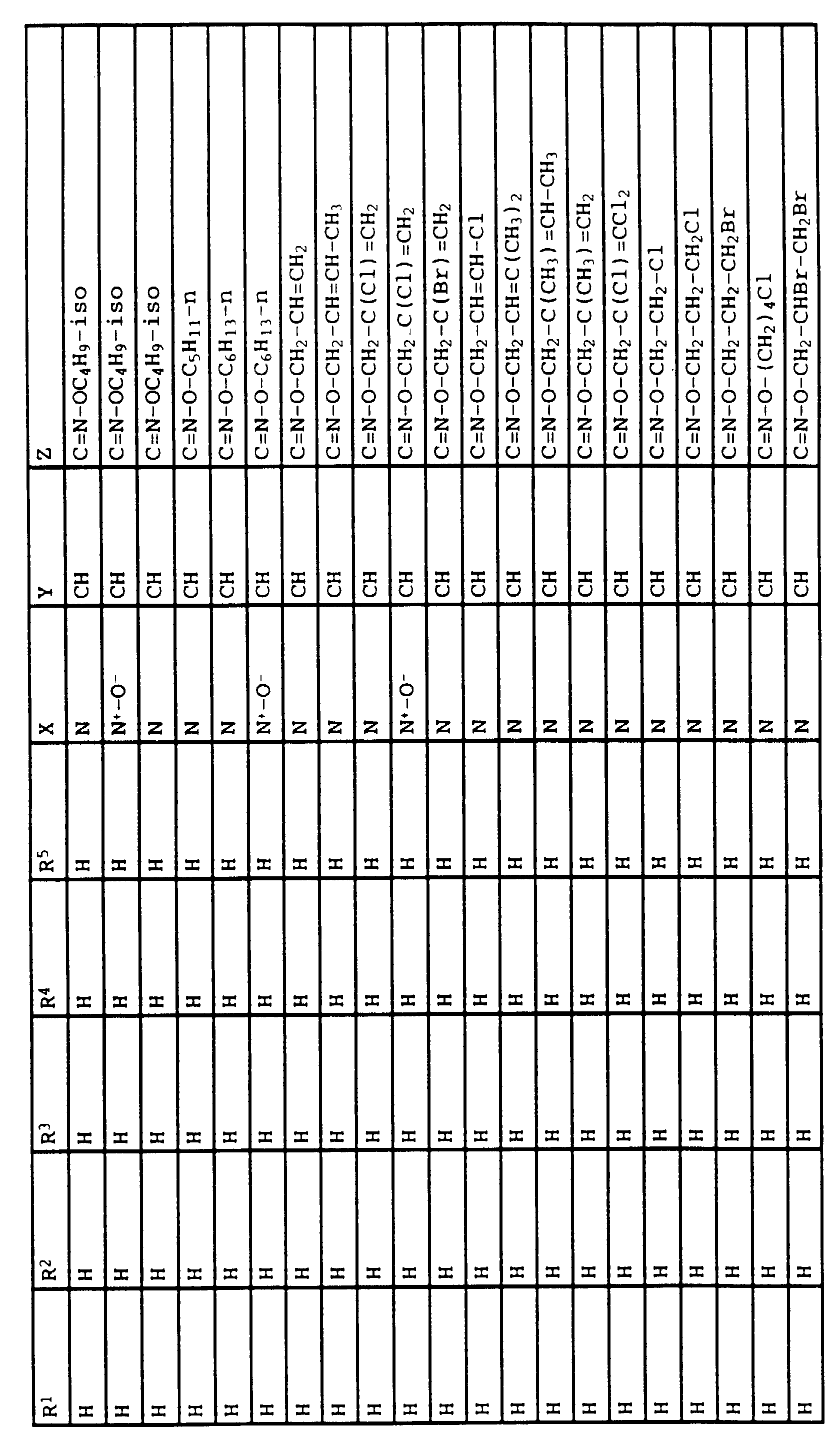

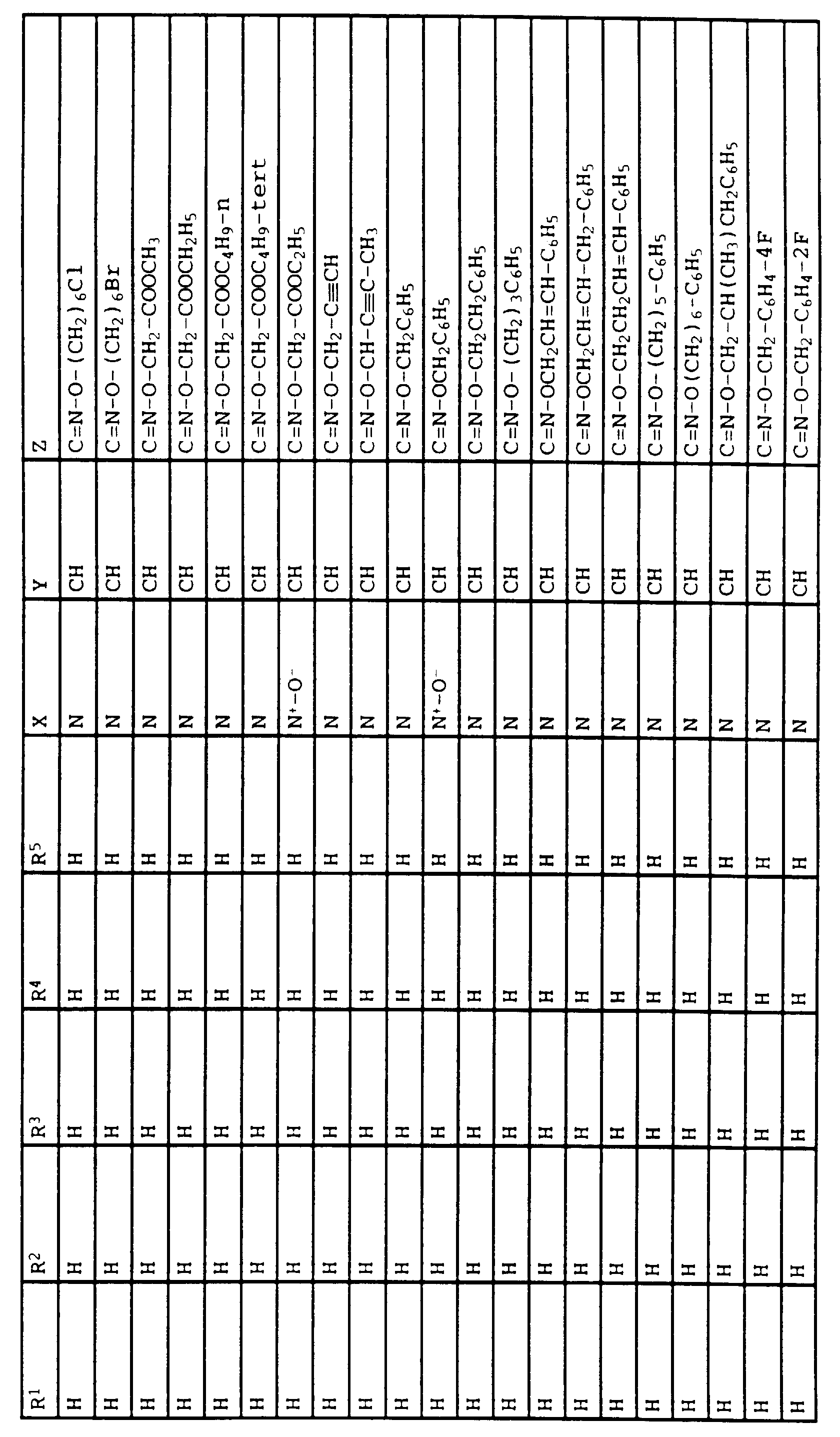

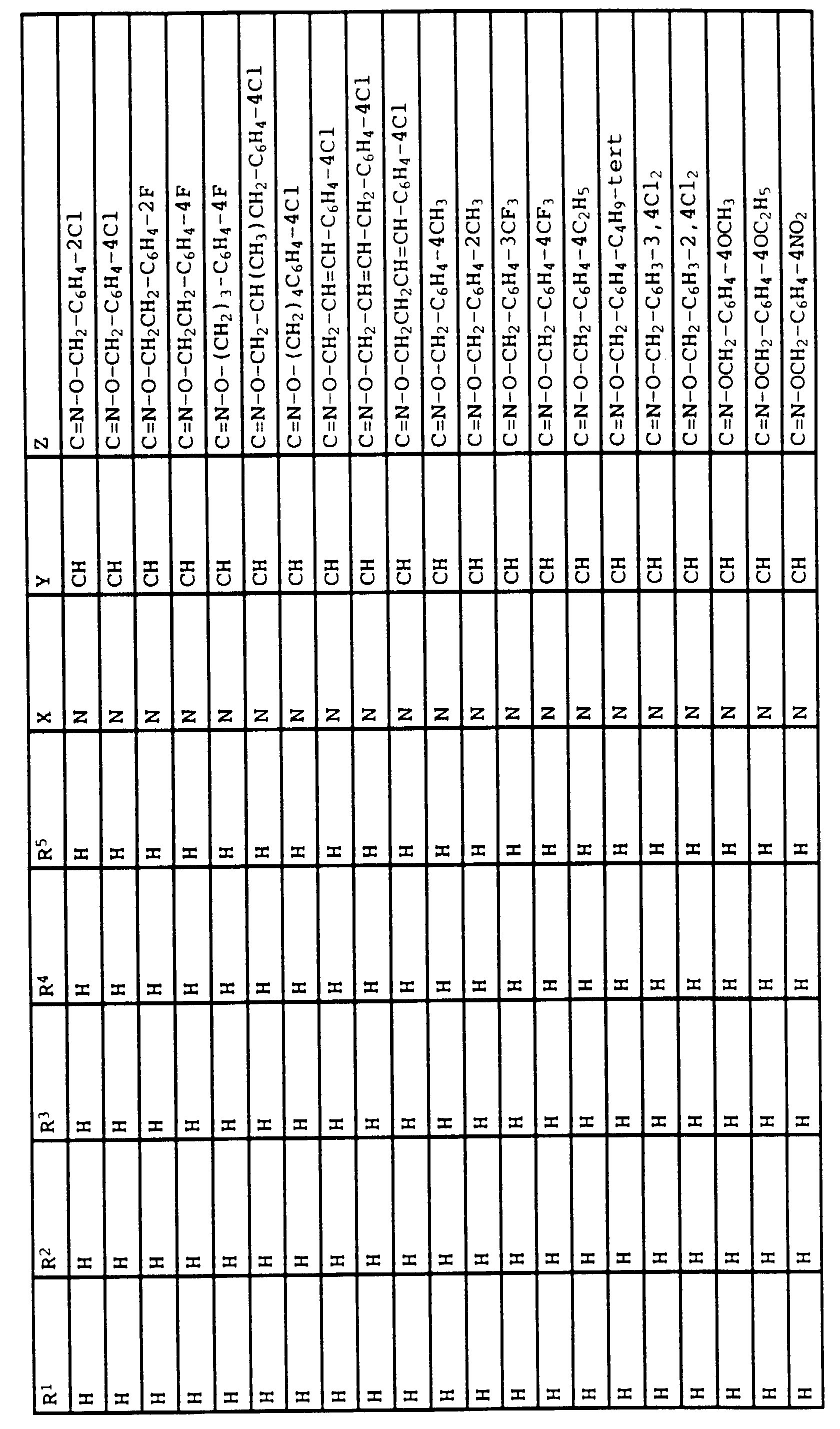

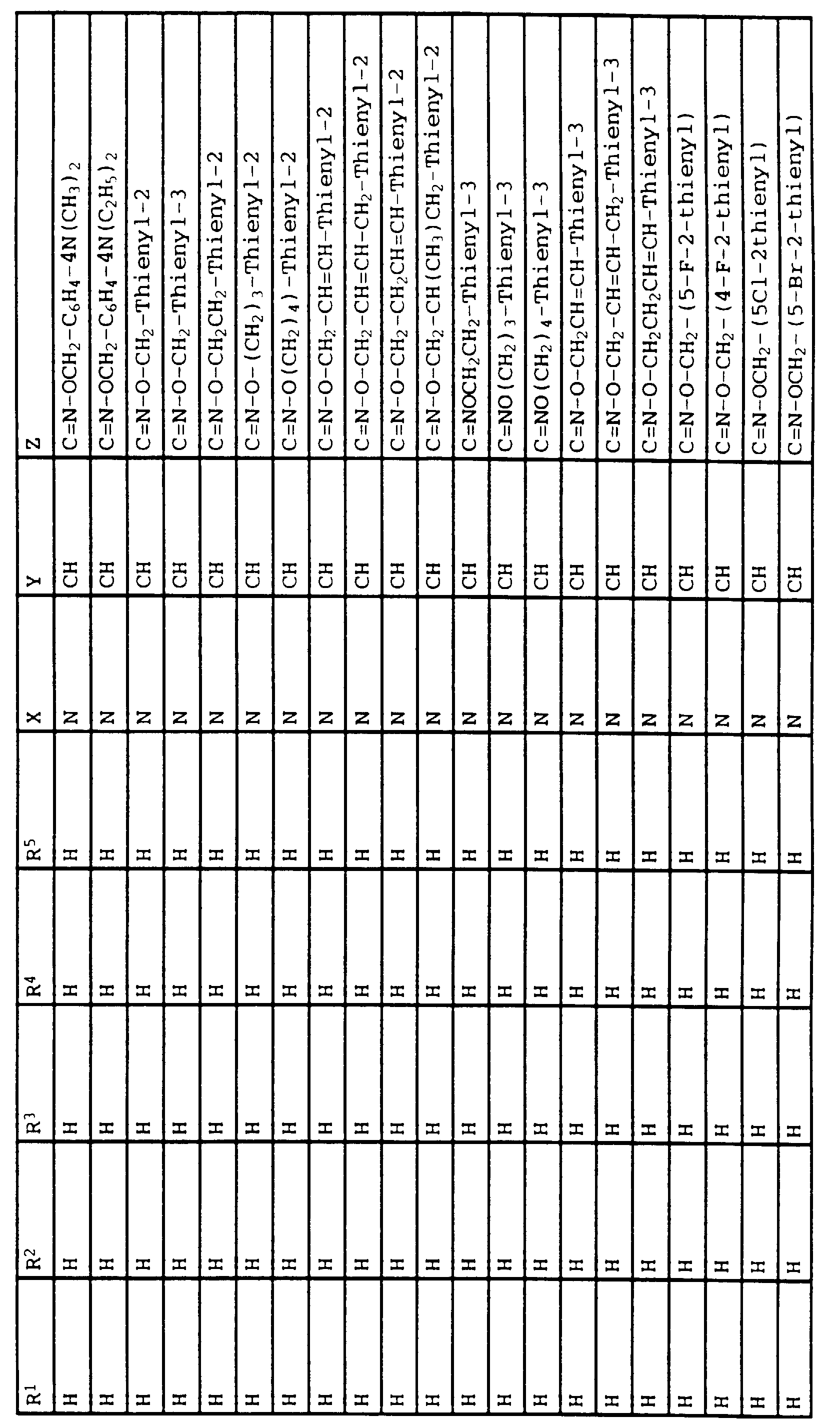

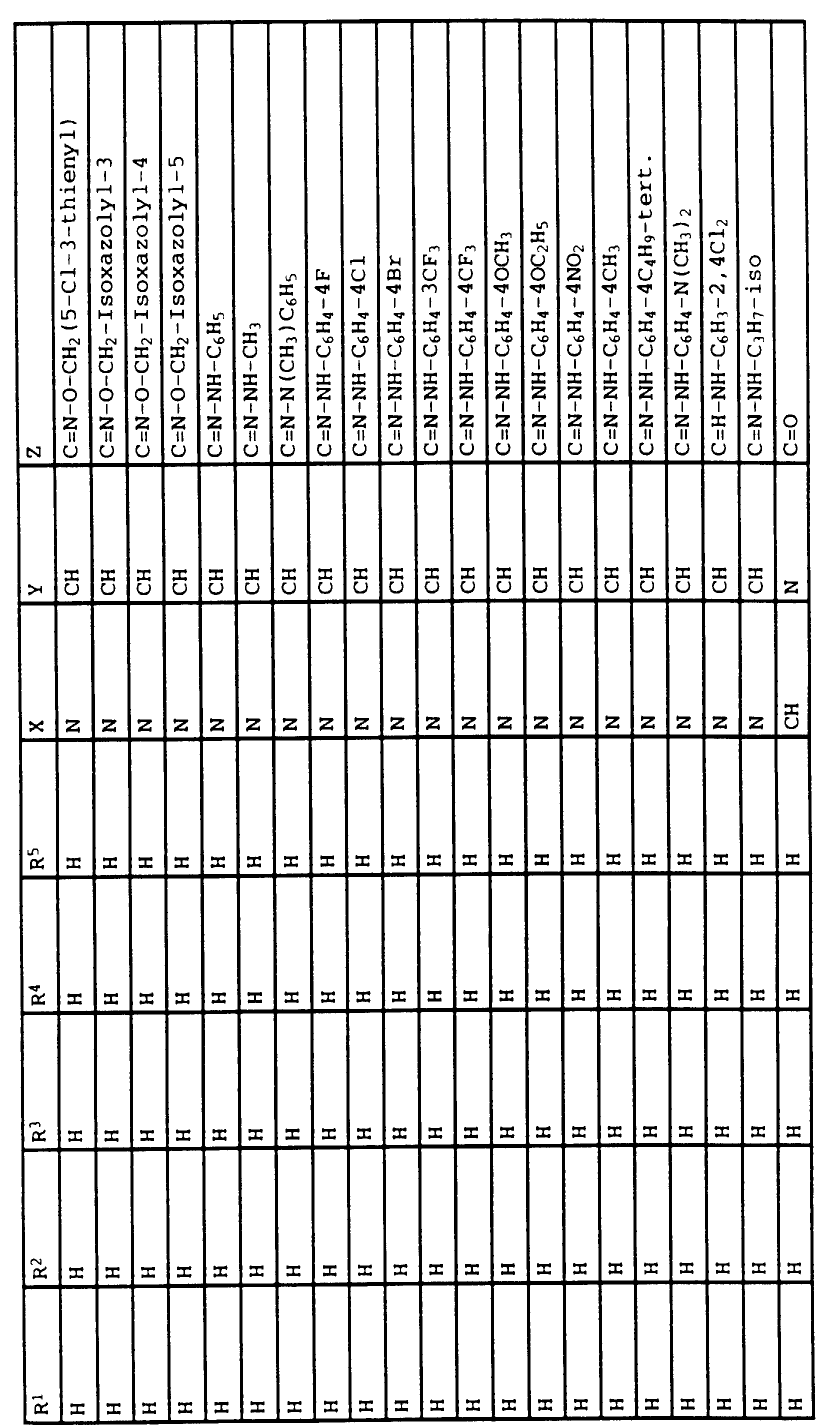

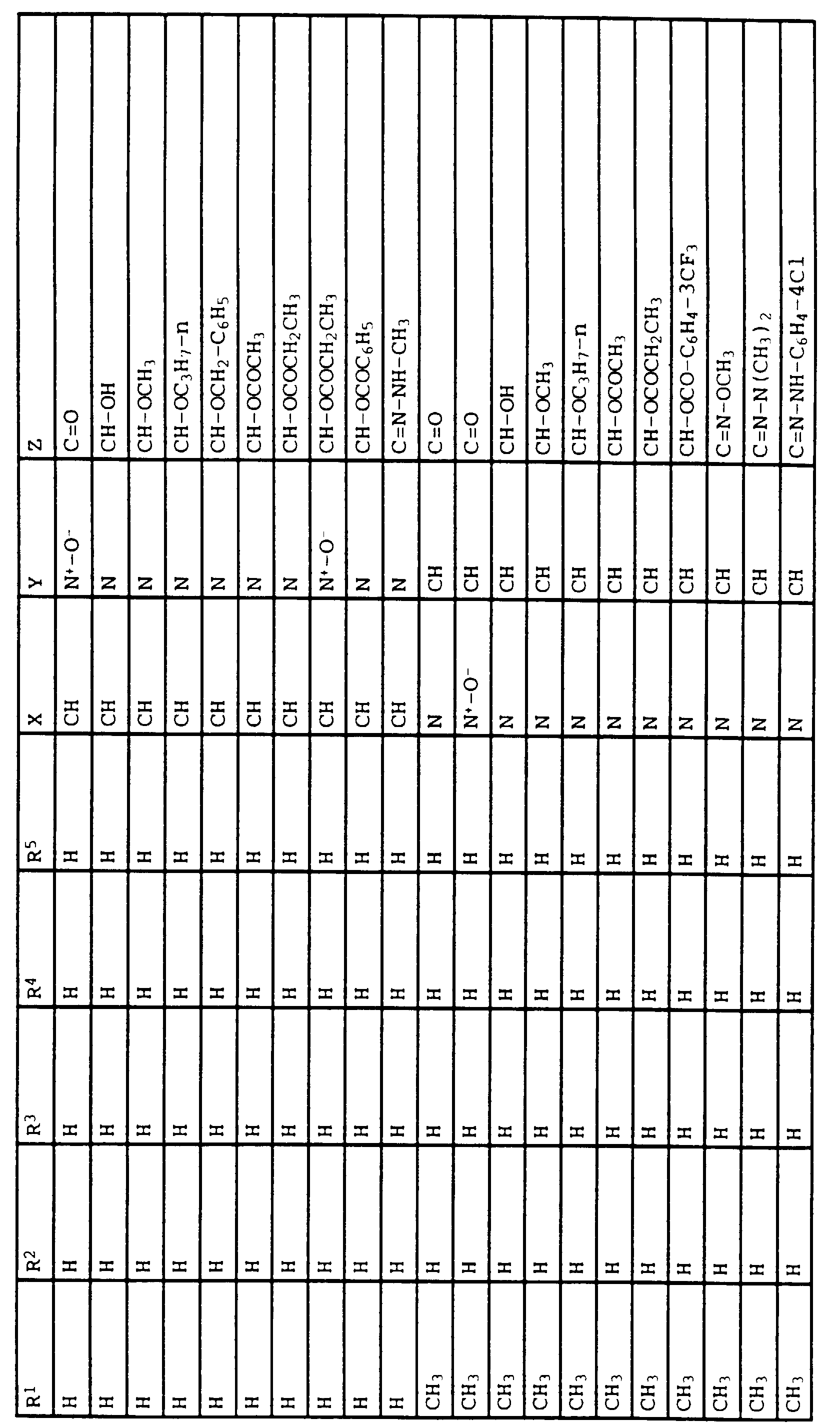

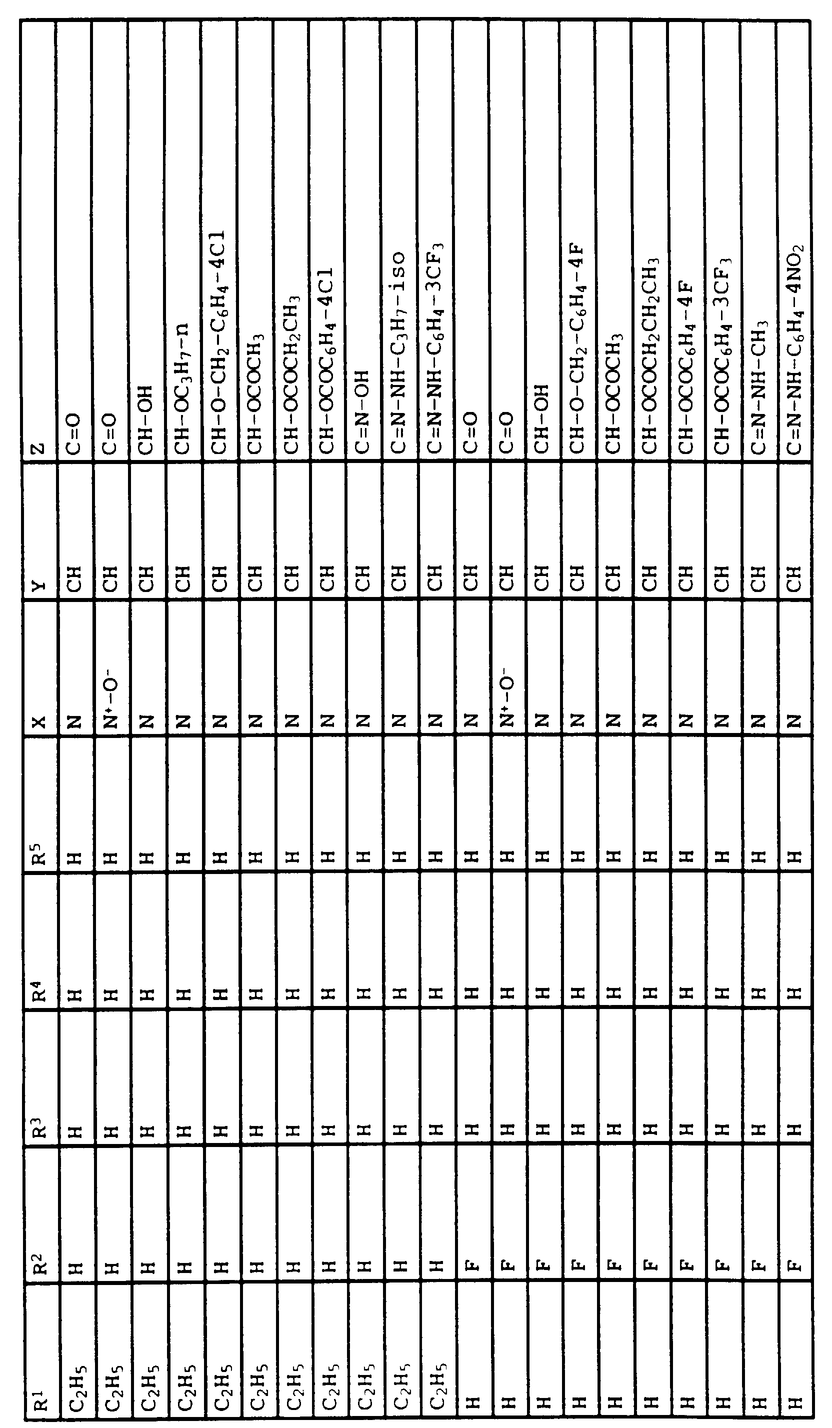

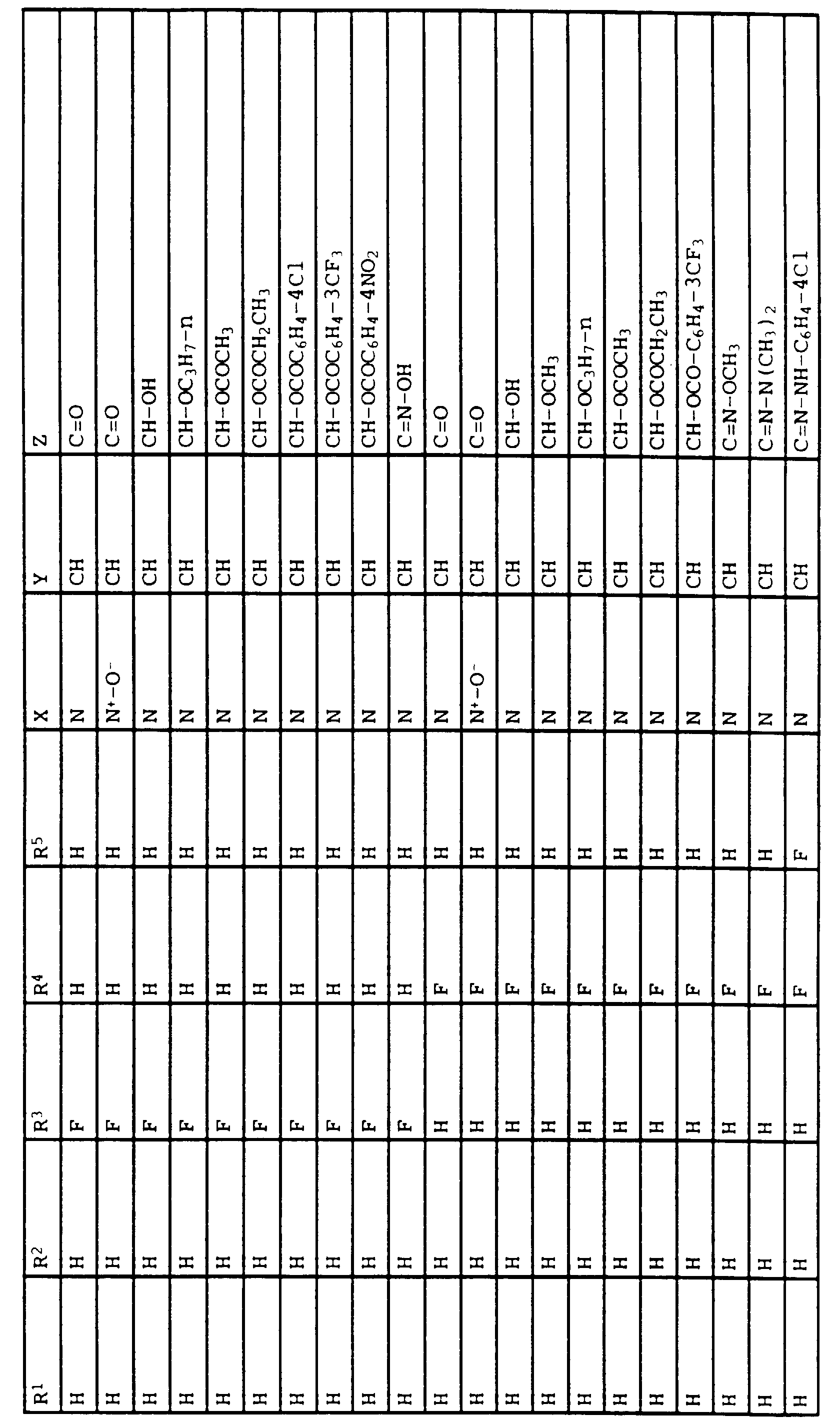

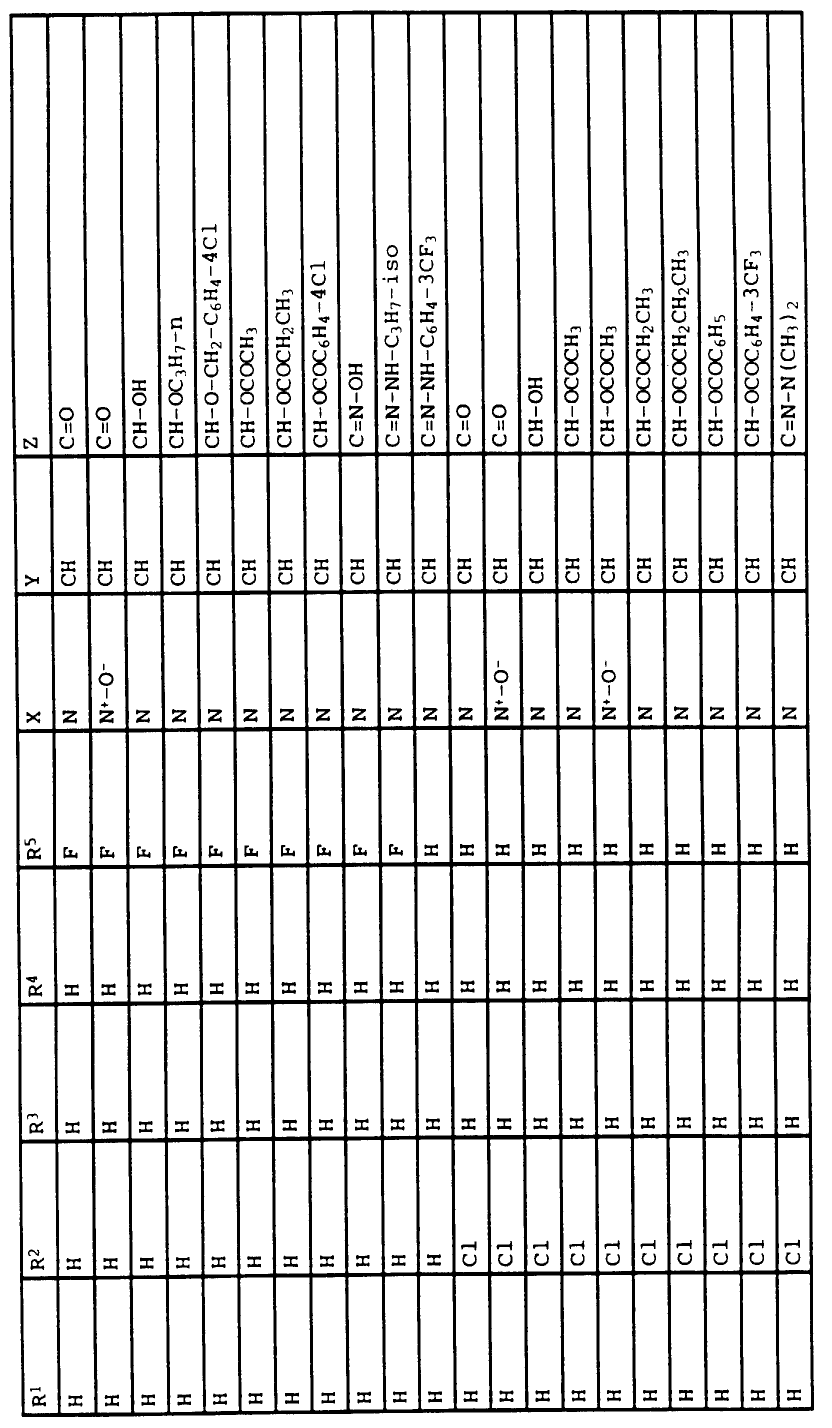

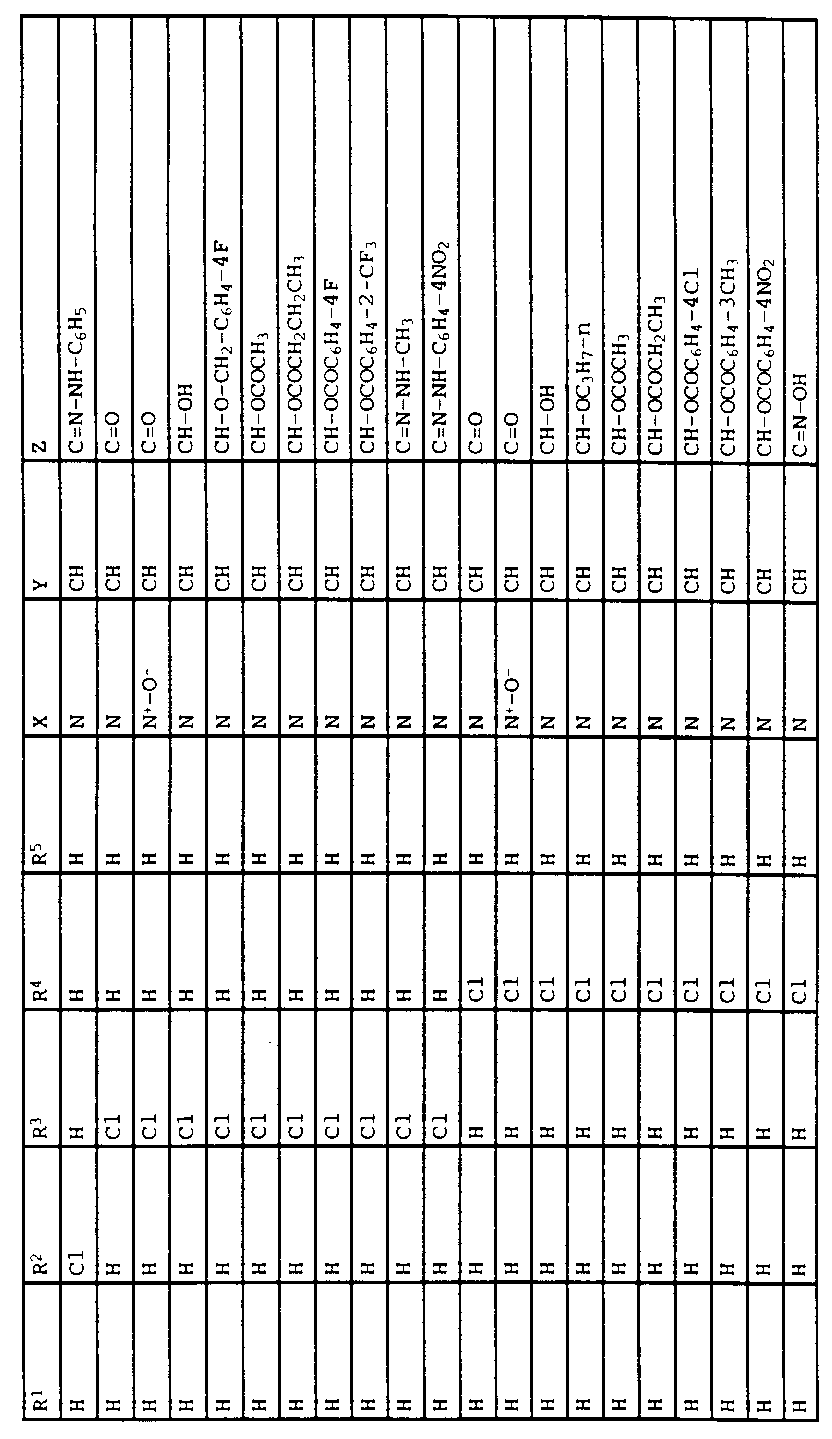

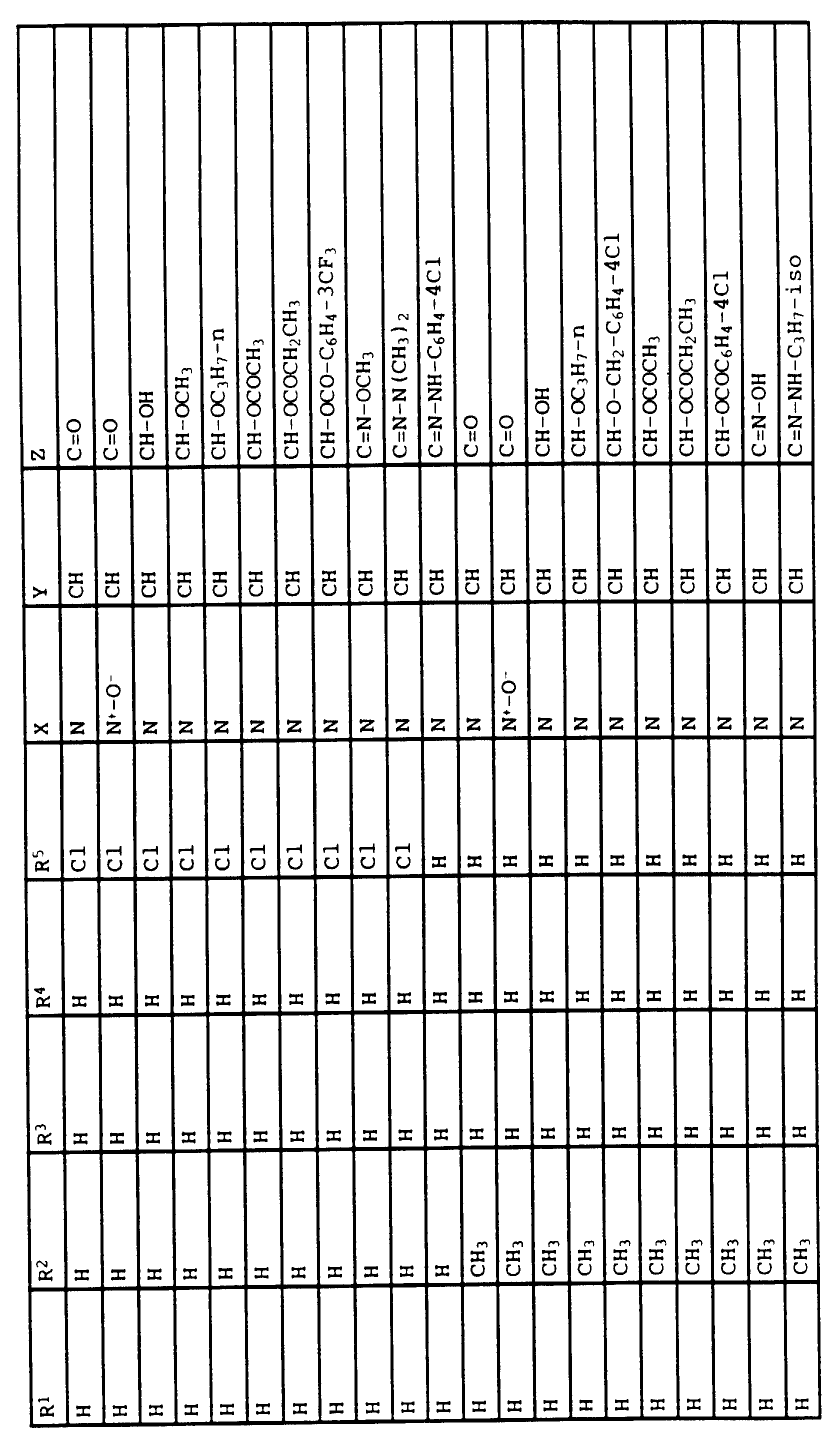

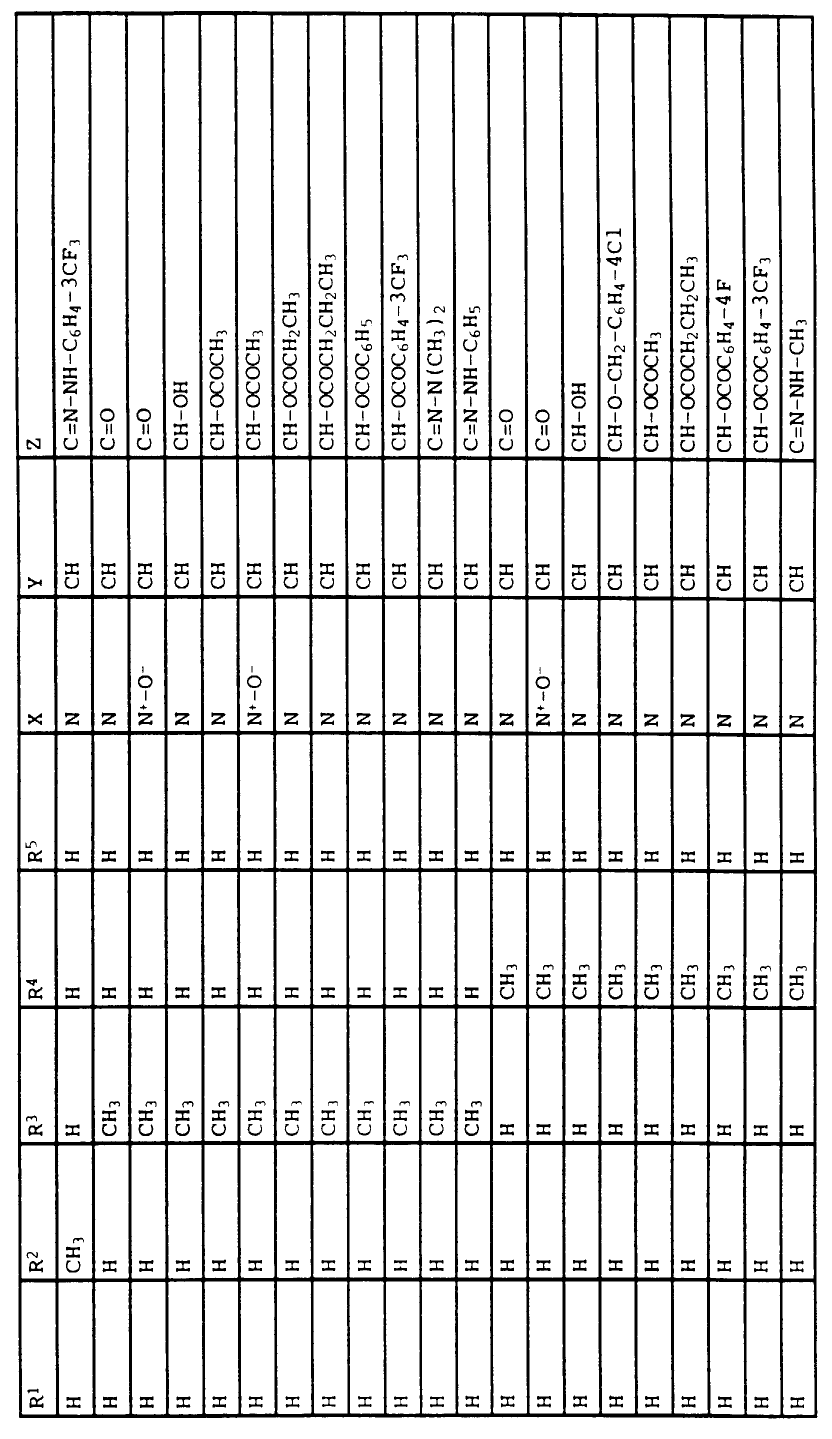

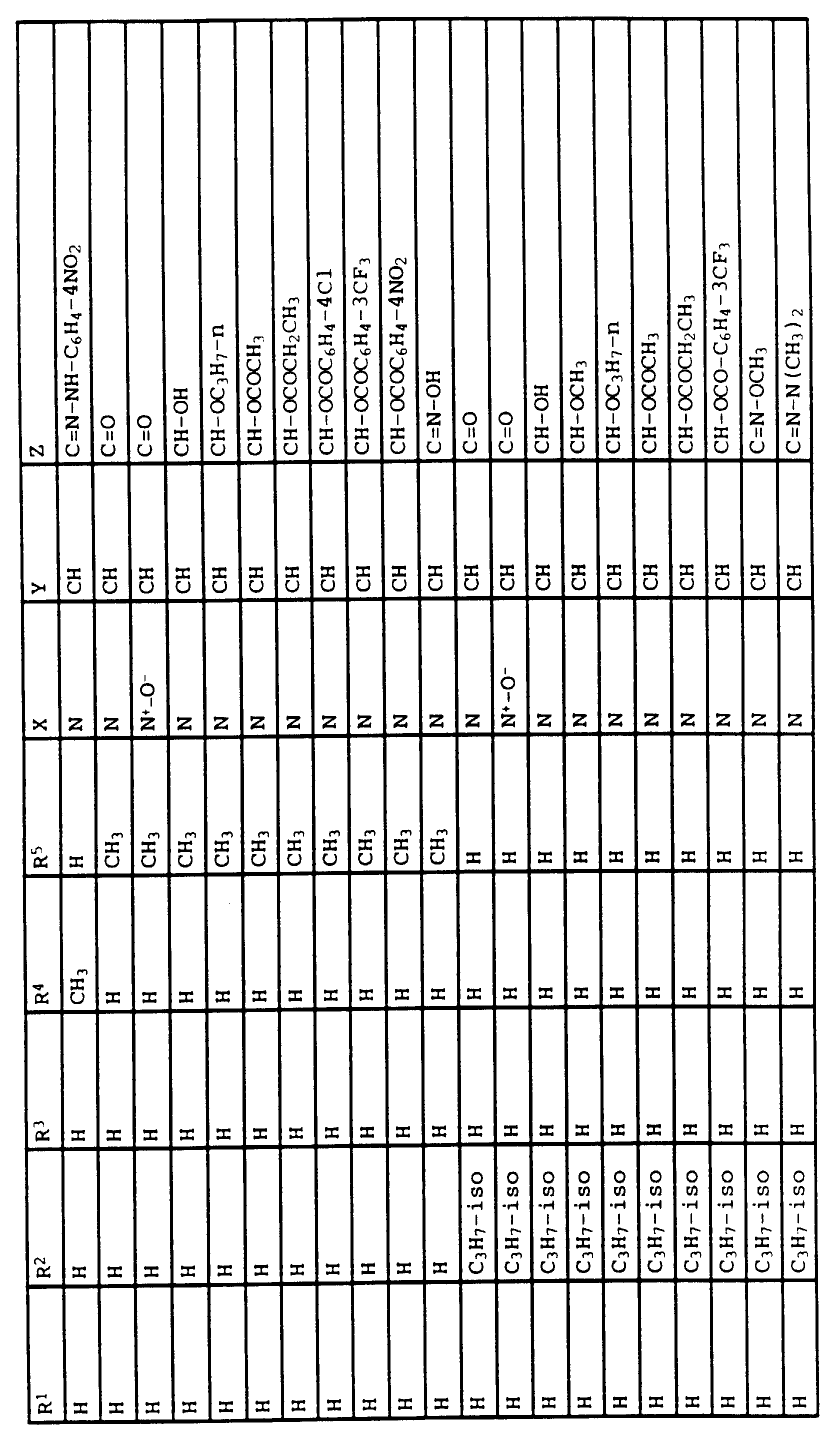

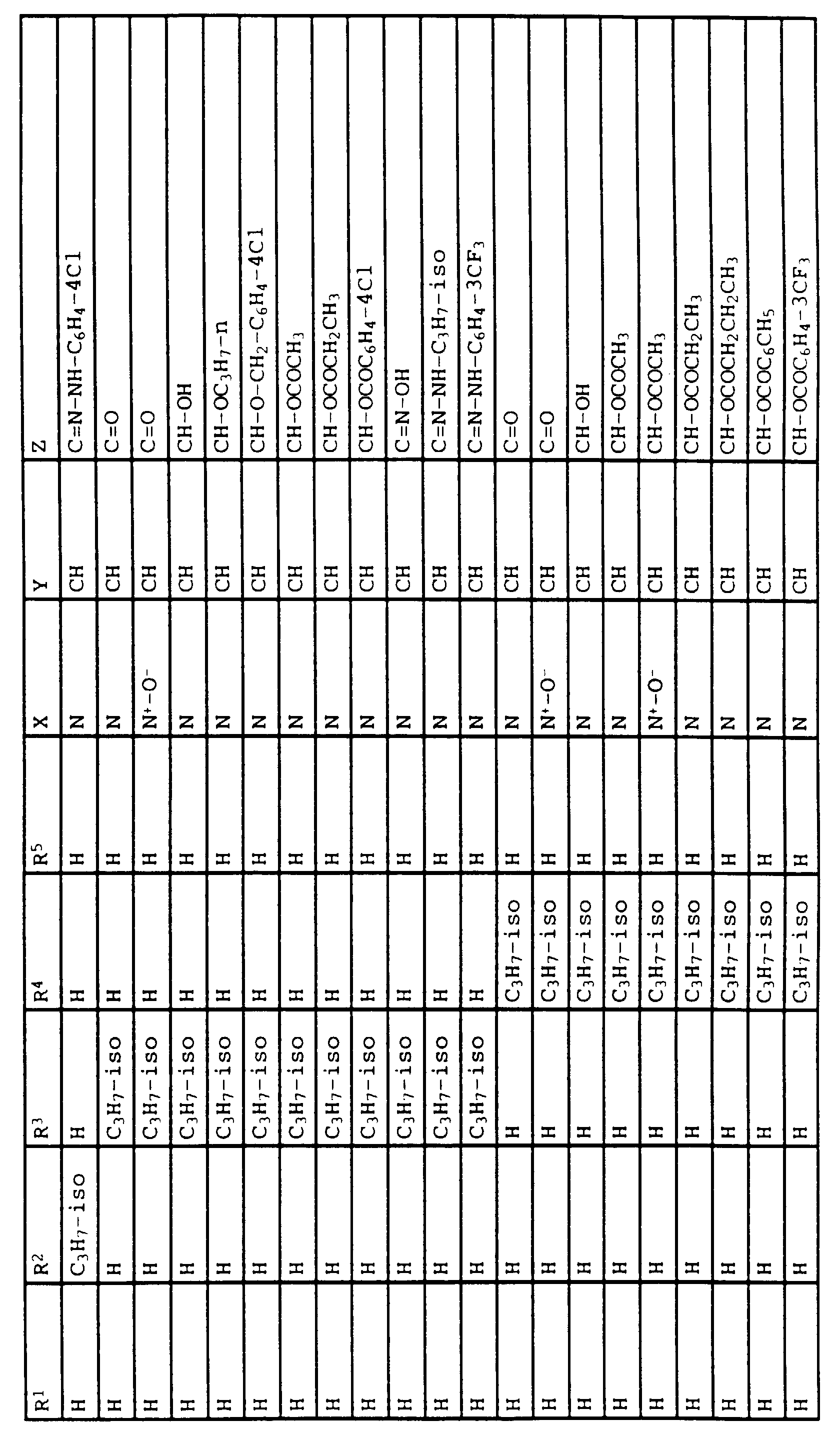

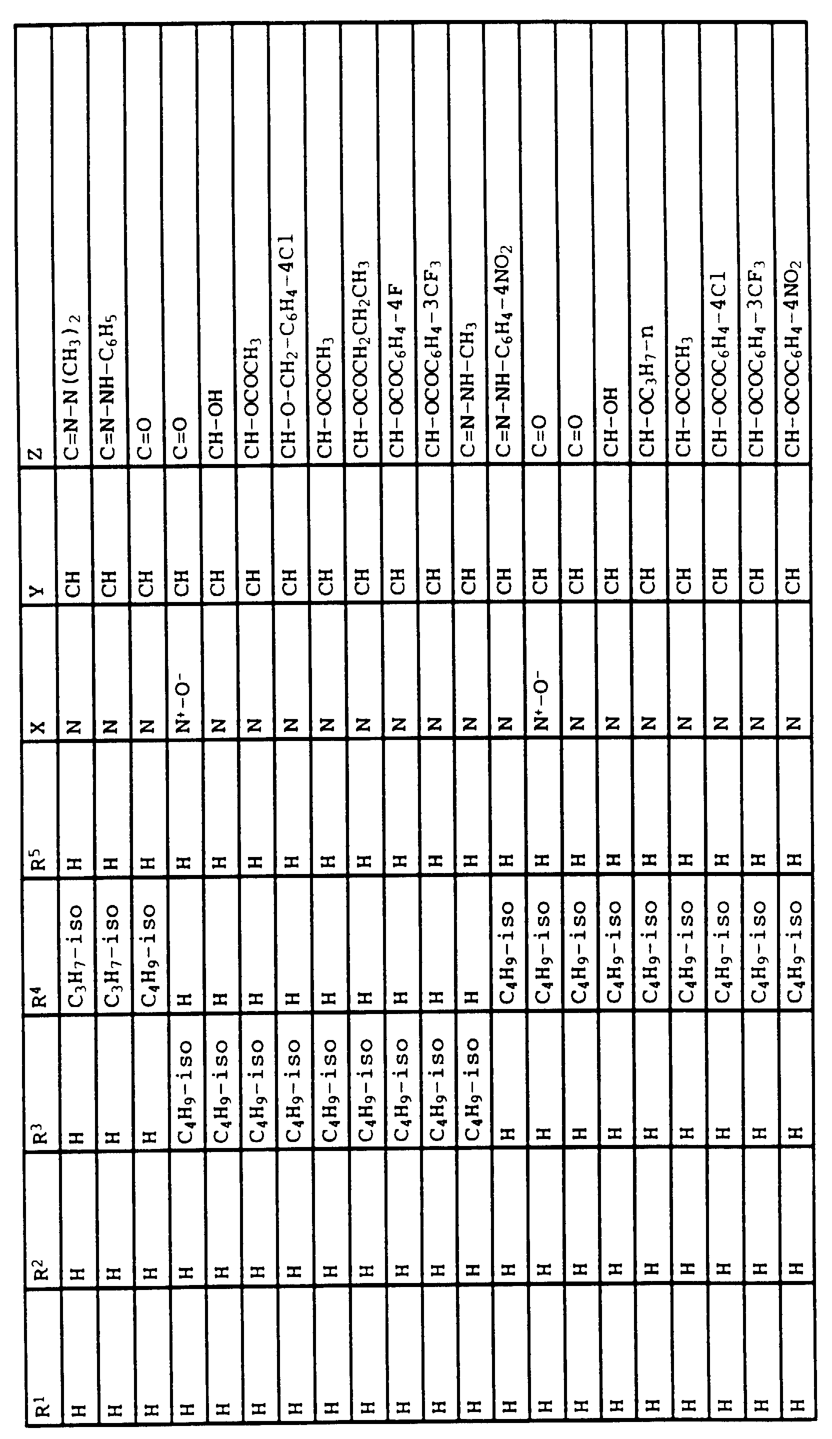

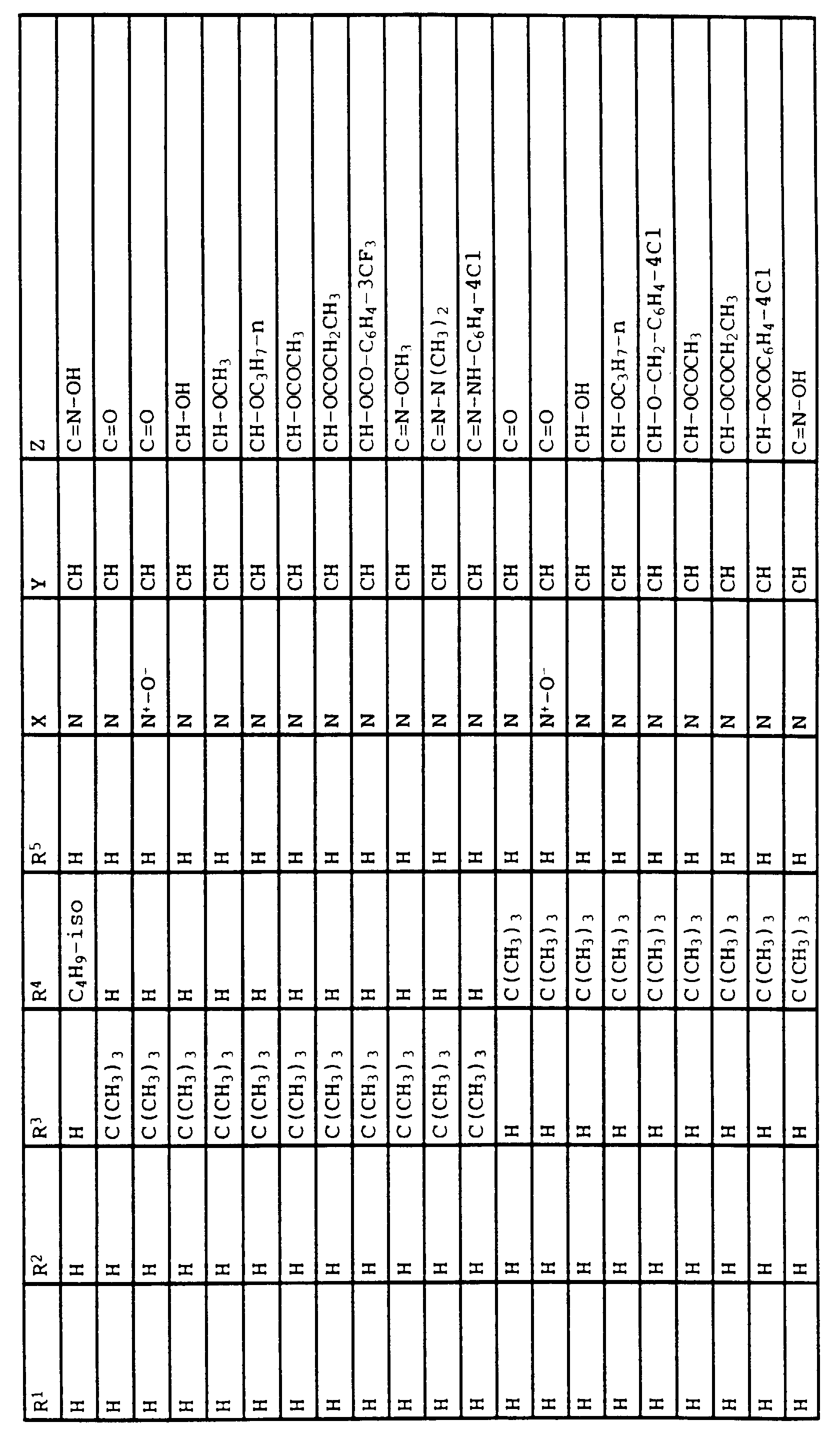

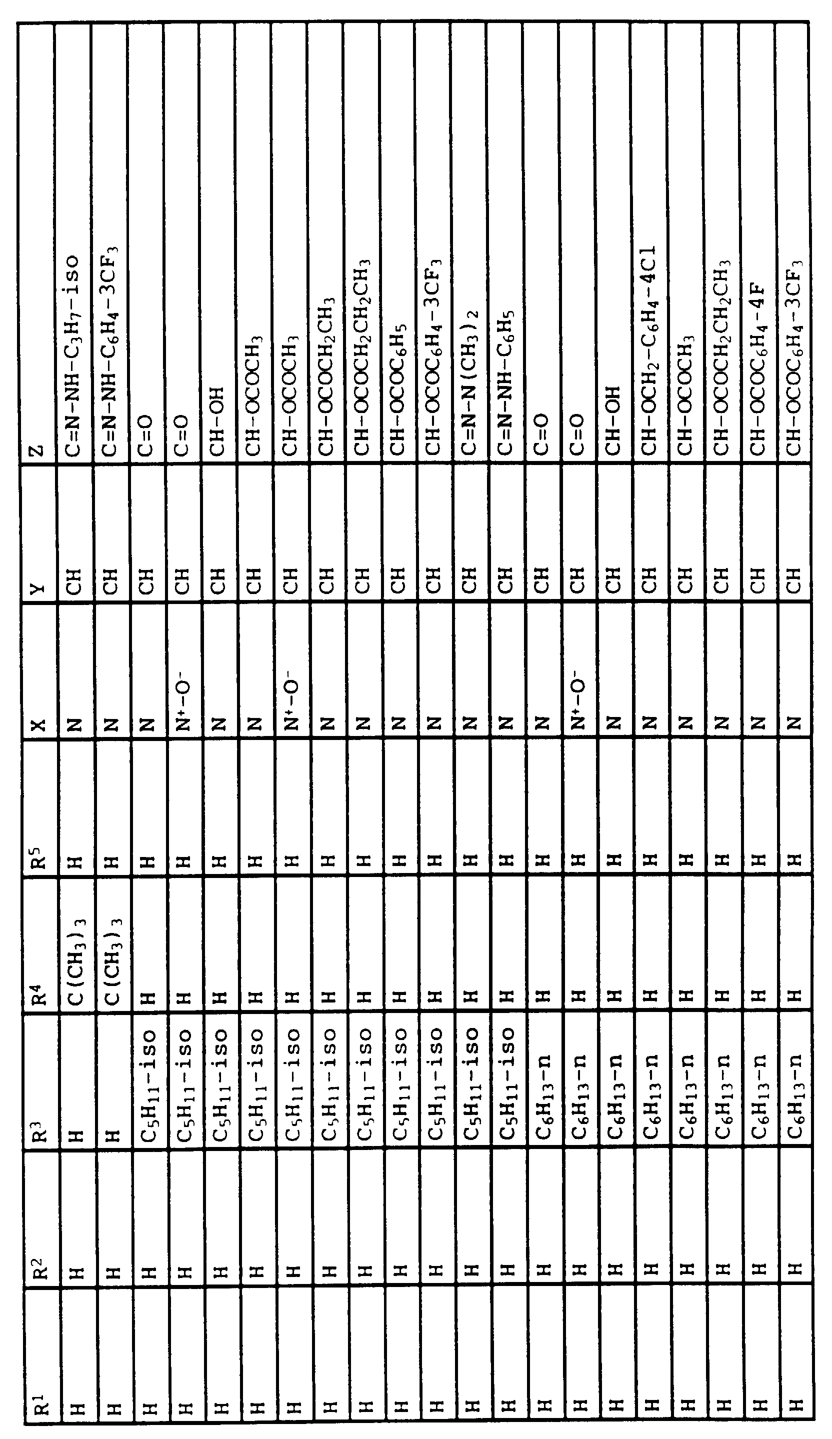

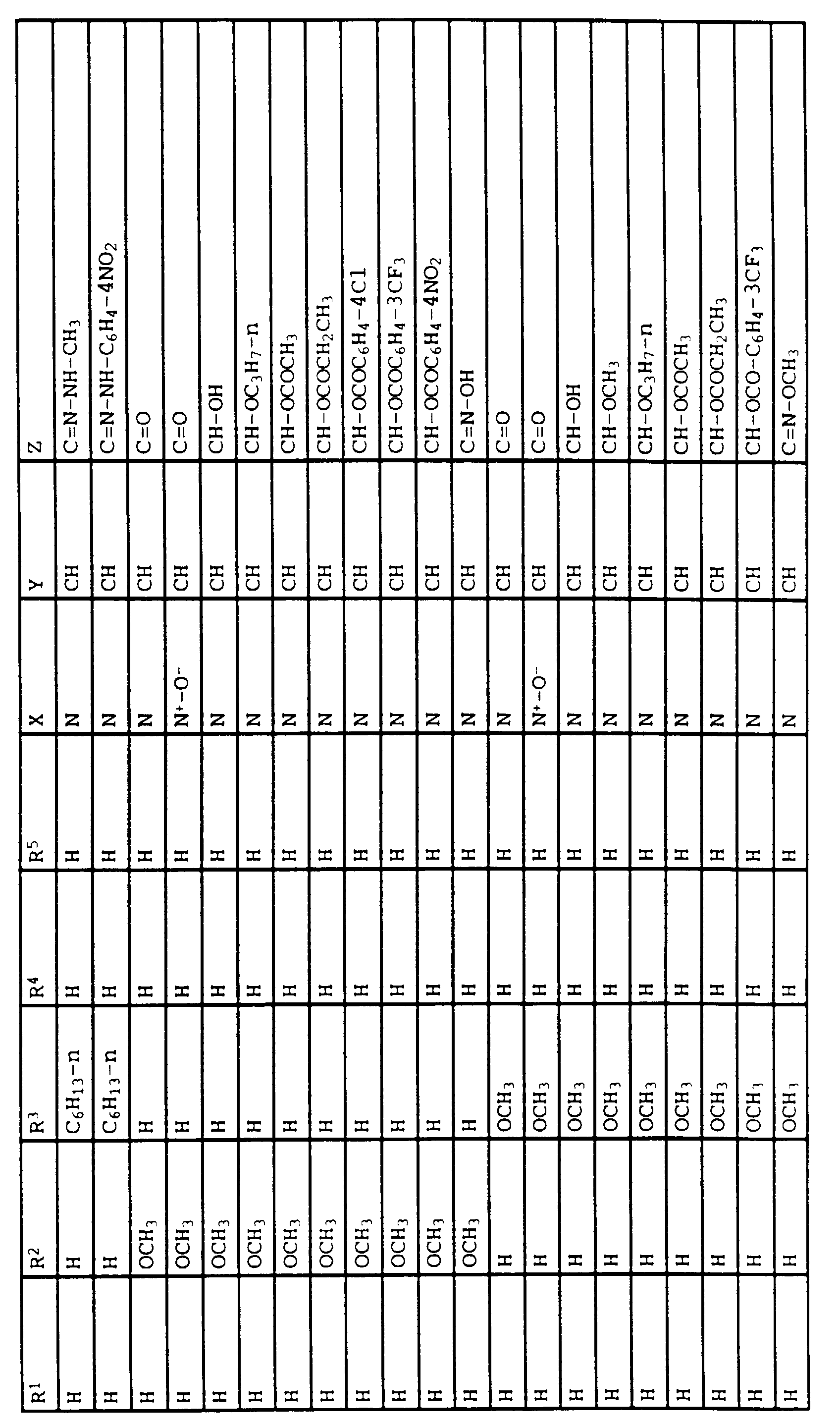

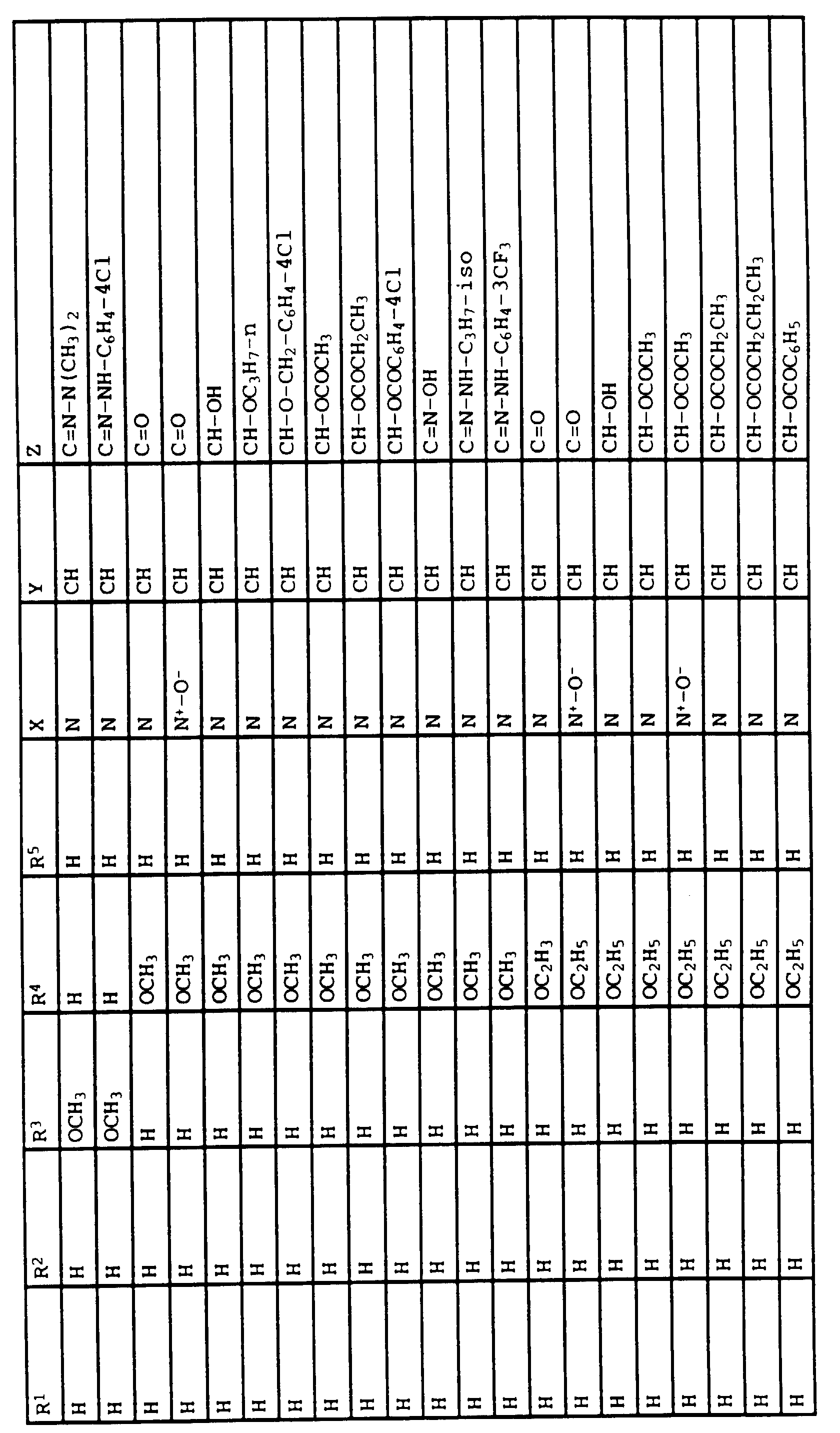

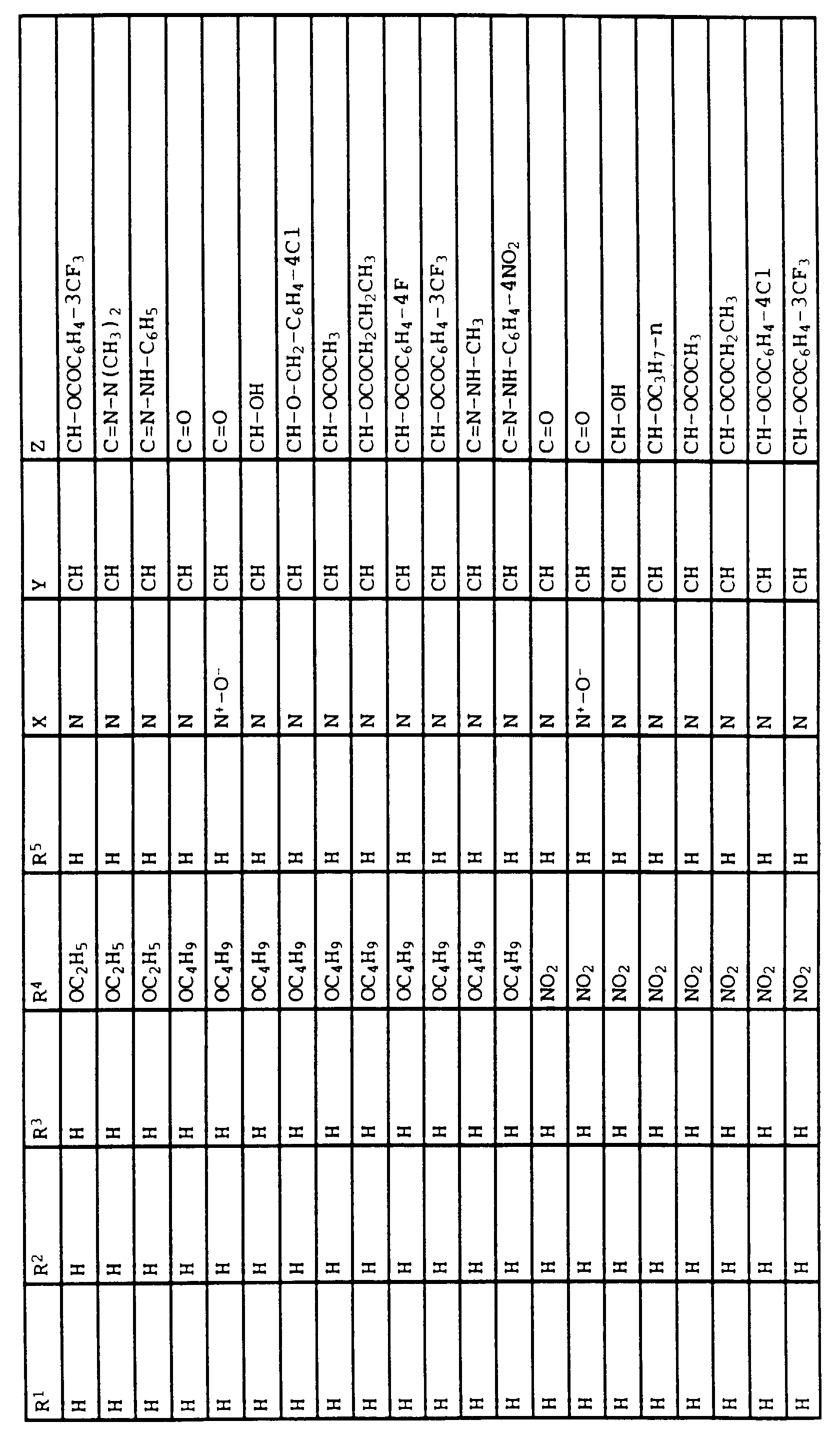

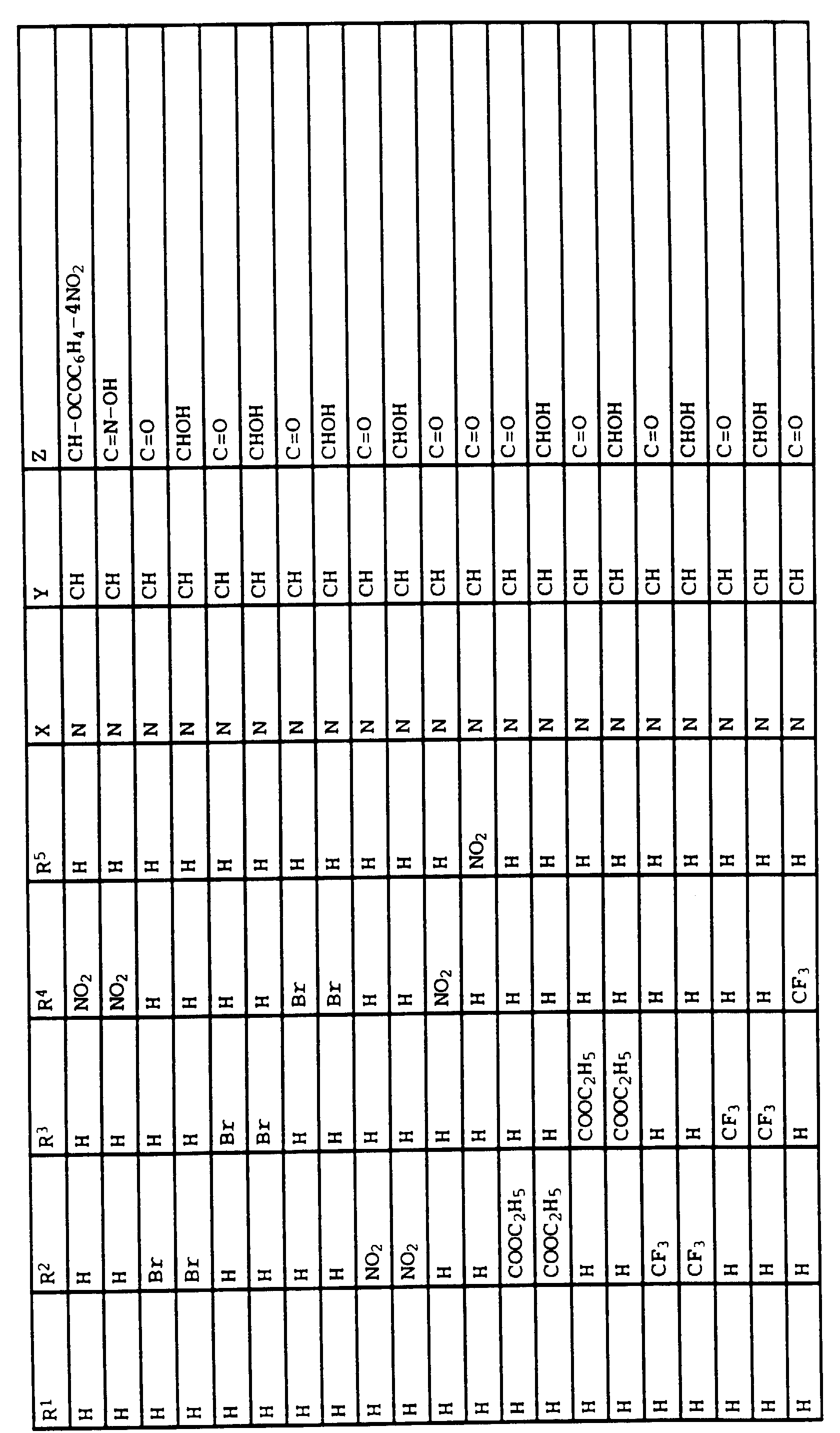

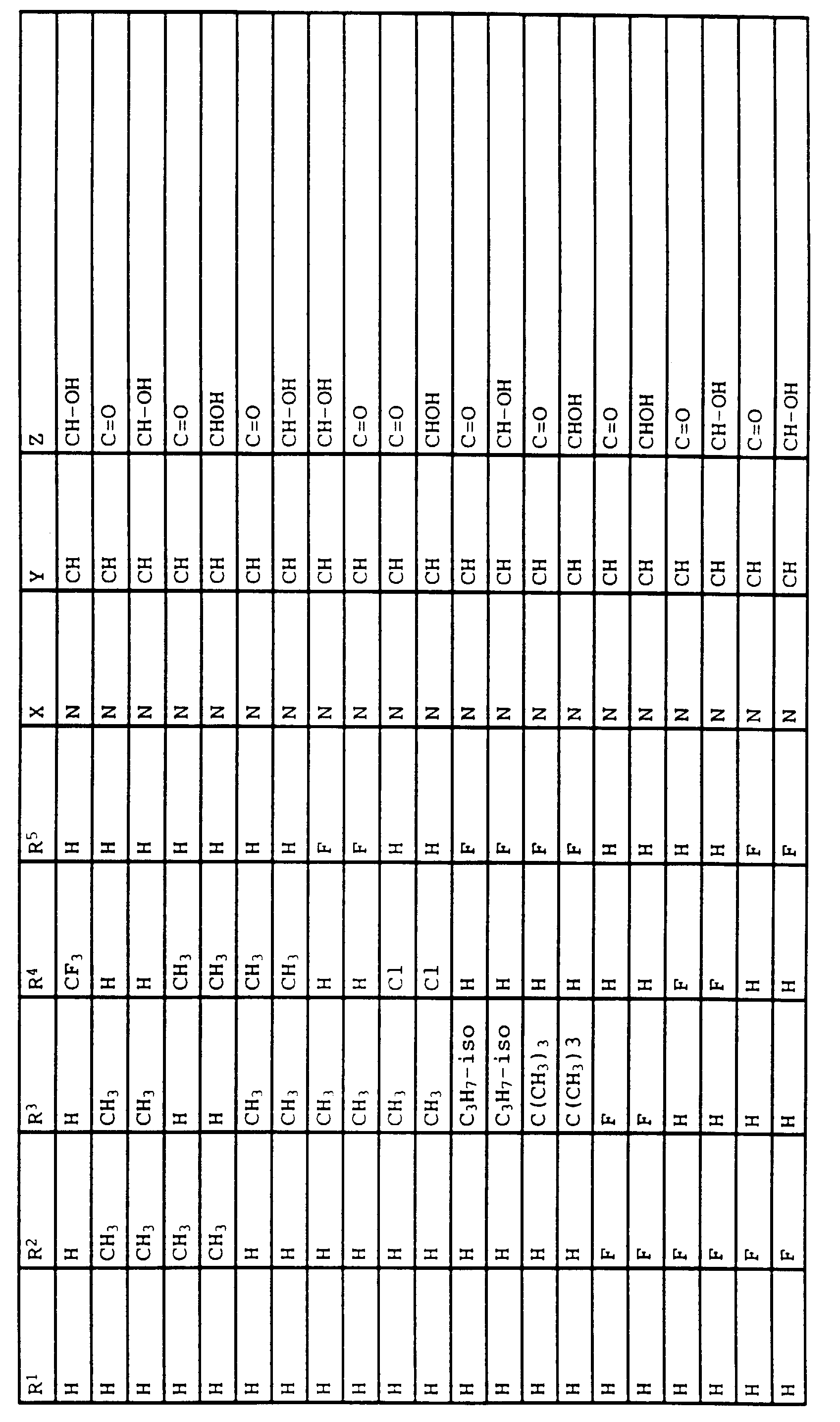

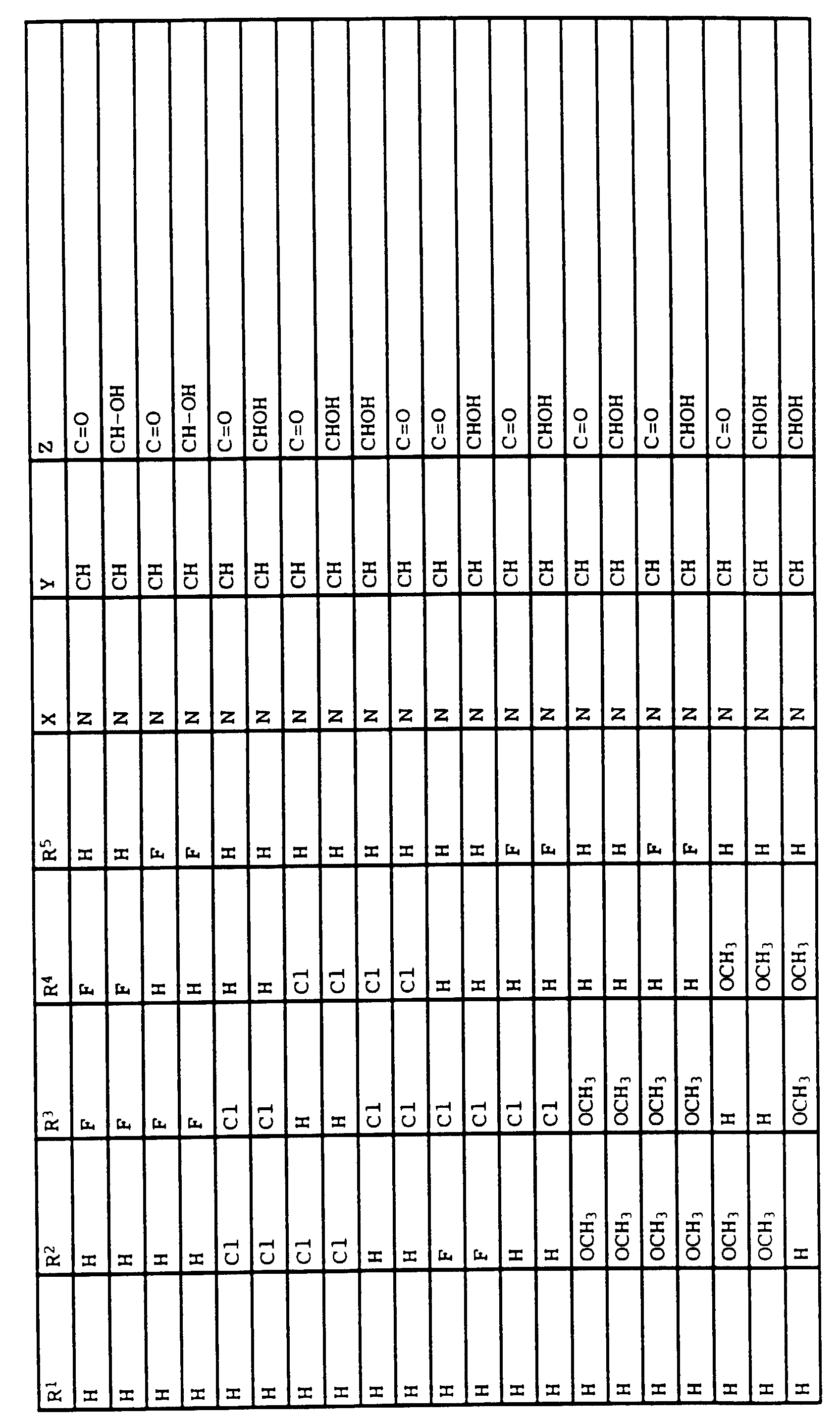

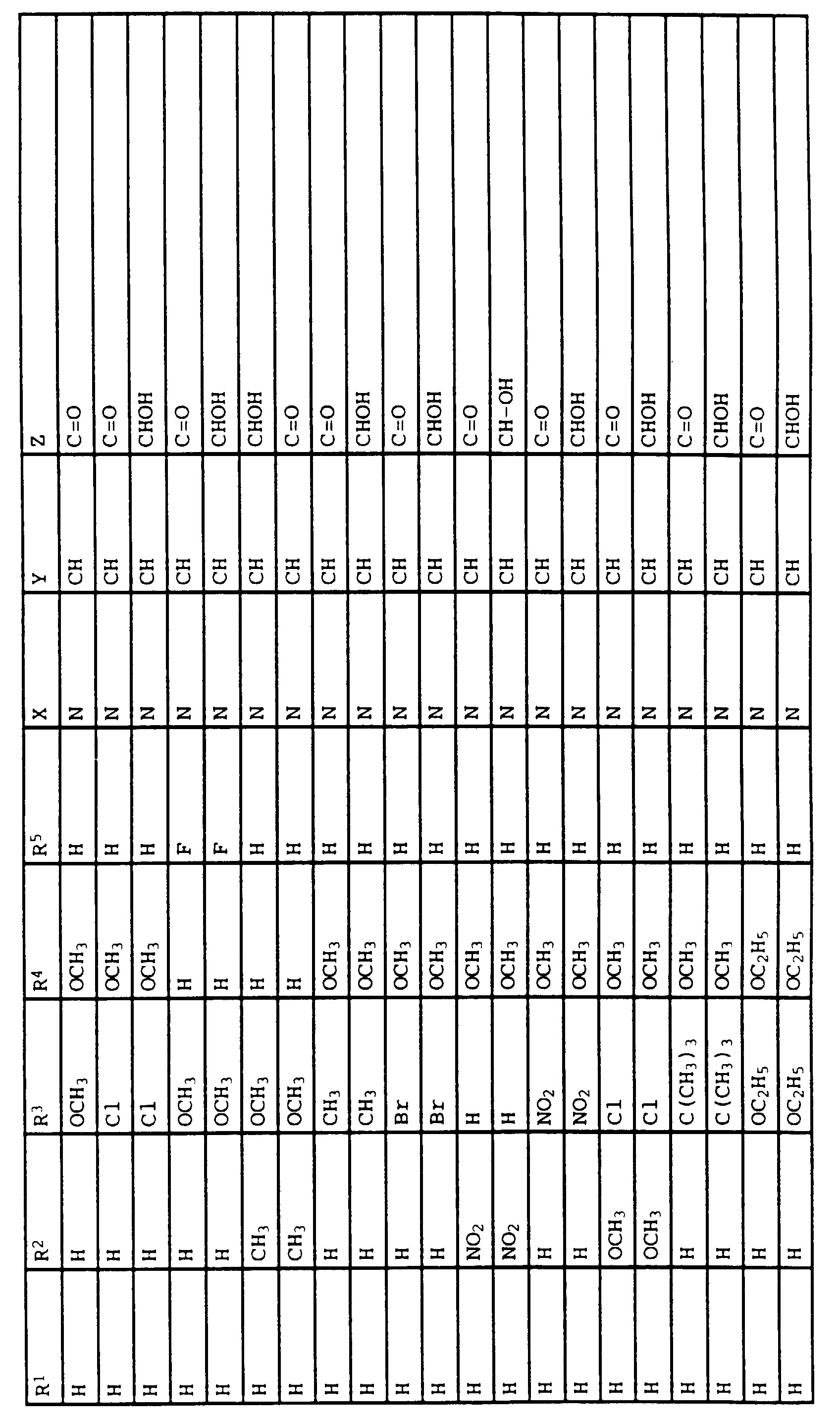

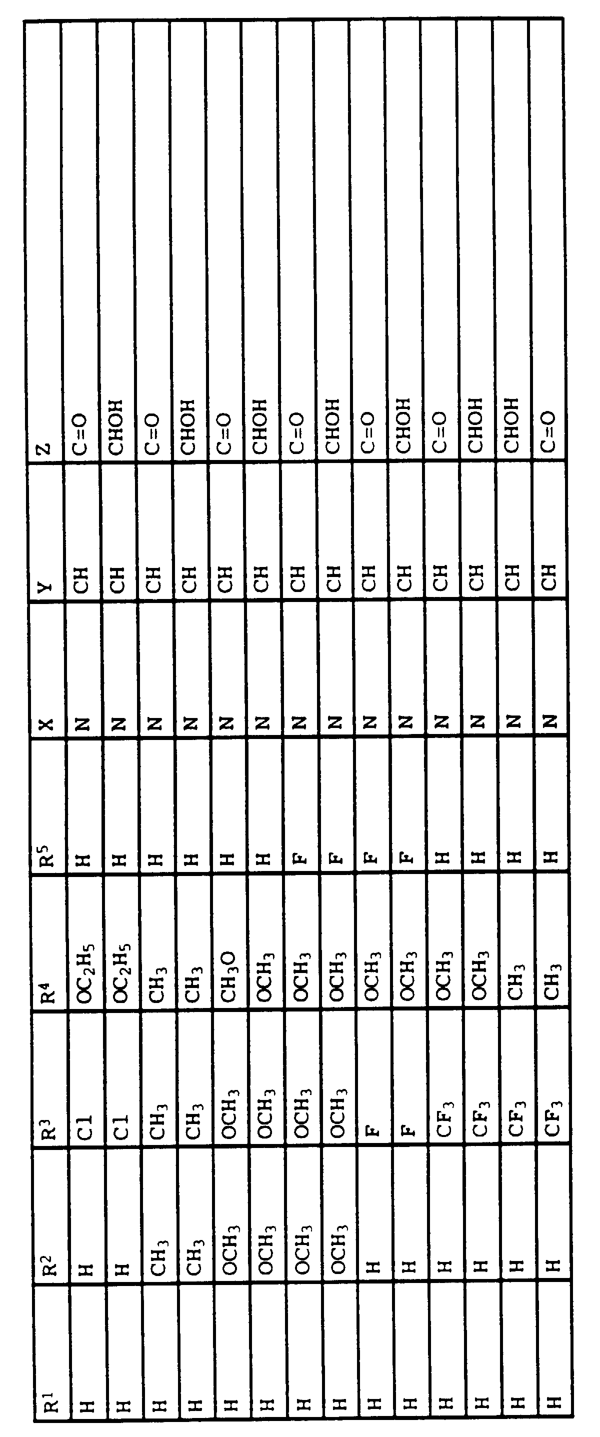

Die Verbindungen I bzw. Ia und die sie enthaltenden herbiziden Mittel sowie deren umweltverträgliche Salze können in Kulturen wie Weizen, Reis, Mais, Soja und Baumwolle Unkräuter und Schadgräser sehr gut bekämpfen, ohne die Kulturpflanzen zu schädigen, ein Effekt, der vor allem auch bei niedrigen Aufwandmengen auftritt.

Die Verbindungen I bzw. Ia und die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I bzw. Ia eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, alkylierte Benzole oder deren Derivate, Methanol, Ethanol, Propanol, Butanol, Cyclohexanol, Cyclohexanon, Chlorbenzol oder stark polare Lösungsmittel, wie N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctyl-phenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR/HPLC/GC-Spektrum) eingesetzt.

Beispiele für solche Zubereitungen sind:
I. 20 Gew.-Teile der Verbindung Nr. 50 werden in einer Mischung gelöst, die aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in 100 000 Gew.-Teilen Wasser erhält man eine wäßrige Dispersion, die 0,02 Gew.-% des Wirkstoffs enthält.
II. 20 Gew.-Teile der Verbindung Nr. 50 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
III. 20 Gew.-Teile des Wirkstoffs Nr. 50 werden in einer Mischung gelöst, die aus 25 Gew.-Teilen Cyclohexanon, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
IV. 20 Gew.-Teile des Wirkstoffs Nr. 50 werden mit 3 Gew.-Teilen des Natriumsalzes der Diisobutylnaphthalin-α-sulfonsäure, 17 Gew.-Teile des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser enthält man eine Spritzbrühe, die 0,1 Gew.-% des Wirkstoffs enthält.
V. 3 Gew.-Teile des Wirkstoffs Nr. 50 werden mit 97 Gew.-Teilen feinteiligem Kaolin vermischt. Man erhält auf diese Weise ein Stäubemittel, das 3 Gew.-% des Wirkstoffs enthält.
VI. 20 Gew.-Teile des Wirkstoffs Nr. 50 werden mit 2 Gew.-Teilen Calciumsalz der Dodecylbenzolsulfonsäure, 8 Gew.-Teilen Fettalkohol-polyglykolether, 2 Gew.-Teilen Natriumsalz eines Phenol-Harnstoff-Formaldehyd-Kondensates und 68 Gew.-Teilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5,0, vorzugsweise 0,01 bis 2,0 kg/ha aktive Substanz (a.S.).

In Anbetracht der Vielseitigkeit der Applikationsmethoden können die erfindungsgemäßen Verbindungen I und I' bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulgaris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum (Gossypium arboreum, Gossypium herbaceum, Gossypium vitifolium), Hevea brasiliensis, Hordeum vulgare, Humulus lupulus, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N. rustica), Olea europaea, Oryza sativa, Phaseolus lunatus, Phaseolus mungo, Phaseolus vulgaris, Phaseolus vulgaris, Picea abies, Pinus spp., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Die wachstumsregulierend wirksamen Verbindungen I bzw. I' können praktisch alle Entwicklungsstadien einer Pflanze verschiedenartig beeinflussen. Die Wirkungsvielfalt der Pflanzenwachstumsregulatoren hängt ab vor allem
a) von der Pflanzenart und -sorte,
b) vom Zeitpunkt der Applikation, bezogen auf das Entwicklungsstadium der Pflanze und von der Jahreszeit,
c) von dem Applikationsort und -verfahren (z.B. Samenbeize, Bodenbehandlung, Blattapplikation oder Stamminjektion bei Bäumen),
d) von klimatischen Faktoren, z.B. Temperatur, Niederschlagsmenge, außerdem auch Tageslänge und Lichtintensität,
e) von der Bodenbeschaffenheit (einschließlich Düngung),
f) von der Formulierung bzw. Anwendungsform des Wirkstoffs und schließlich
g) von der angewendeten Konzentration der aktiven Substanz.

Aus der Reihe der verschiedenartigen Anwendungsmöglichkeiten der Pflanzenwachstumsregulatoren der Formel I bzw. I' im Pflanzenanbau, in der Landwirtschaft und im Gartenbau, werden einige nachstehend erwähnt.
A. Mit den erfindungsgemäß verwendbaren Verbindungen läßt sich das vegetative Wachstum der Pflanzen stark hemmen, was sich insbesondere in einer Reduzierung des Längenwachstums äußert.
   Die behandelten Pflanzen weisen demgemäß einen gedrungenen Wuchs aus; außerdem ist eine dunklere Blattfärbung zu beobachten.
   Als vorteilhaft für die Praxis erweist sich eine verminderte Intensität des Wachstums von Gräsern sowie lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.
   Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.
   Bei Obst- und anderen Bäumen lassen sich mit den Wachstumsregulatoren Schnittkosten einsparen. Außerdem kann die Alternanz von Obstbäumen durch Wachstumsregulatoren gebrochen werden.
   Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.
   Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generative Phase neigen. Auch bei anderen Kulturen, z.B. Wintergetreide, ist es vorteilhaft, wenn die Bestände durch Behandlung mit den erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt bzw. Pflanzenmasse - dem Befall mit verschiedenen Krankheiten (z.B. Pilzkrankheit) vorgebeugt werden.
B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Citrusfrüchten zu erhöhen, den Proteingehalt in Getreide oder Soja zu steigern oder Gummibäume zum vermehrten Latexfluß zu stimulieren.
   Dabei können die Verbindungen der Formel I bzw. I' Ertragssteigerungen durch Eingriffe in den pflanzlichen Stoffwechsel bzw. durch Förderung oder Hemmung des vegetativen und/oder des generativen Wachstums verursachen.
C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.
   Von wirtschaftlichem Interesse ist beispielsweise die Ernteerleichterung, die durch das zeitlich konzentrierte Abfallen oder Vermindern der Haftfestigkeit am Baum bei Citrusfrüchten, Oliven oder bei anderen Arten und Sorten von Kern-, Stein- und Schalenobst ermöglicht wird. Derselbe Mechanismus, d.h. die Förderung der Ausbildung von Trenngewebe zwischen Frucht-, bzw. Blatt- und Sproßteil der Pflanze ist auch für ein gut kontrollierbares Entblättern von Nutzpflanzen wie beispielsweise Baumwolle wesentlich.
D. Mit Wachstumsregulatoren kann weiterhin der Wasserverbrauch von Pflanzen reduziert werden. Durch den Einsatz der erfindungsgemäßen Substanzen läßt sich die Intensität der Bewässerung reduzieren und damit eine kostengünstigere Bewirtschaftung durchführen, weil u.a. die Öffnungsweite der Stomata reduziert wird, eine dickere Epidermis und Cuticula ausgebildet werden, die Durchwurzelung des Bodens verbessert wird und das Mikroklima im Pflanzenbestand durch einen kompakteren Wuchs günstig beeinflußt wird.

Besonders gut eignen sich die Verbindungen I bzw. I' zur Halmverkürzung von Kulturpflanzen wie Gerste, Raps und Weizen.

Die erfindungsgemäß zu verwendenden Wirkstoffe der Formel I bzw. I' können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Die Aufwandmenge an Wirkstoff ist infolge der hohen Pflanzenverträglichkeit nicht kritisch. Die optimale Aufwandmenge variiert je nach Bekämpfungsziel, Jahreszeit, Zielpflanzen und Wachstumsstadien.

Bei der Saatgutbehandlung werden im allgemeinen Wirkstoffmengen von 0.001 bis 50 g, vorzugsweise 0.01 bis 10 g, je Kilogramm Saatgut benötigt.

Für die Blatt- und Bodenbehandlung sind im allgemeinen Gaben von 0.001 bis 10 kg/ha, insbesondere 0.01 bis 3 kg/ha als ausreichend zu betrachten.

Zur Verbreiterung des Wirkungsspektrums zur Erzielung synergistischer Effekte können die Indenopyridine der allgemeinen Formel I bzw. I' mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, die in 2-Stellung z.B. eine Carboxy- oder Carbimino-Gruppe tragen, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I bzw. I' allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Die Herstellung und die Verwendung der Wirkstoffe I bzw. I' geht aus den nachfolgenden Beispielen hervor.

### Herstellungsbeispiele

### Beispiel 1

a) 4-Methyl-5H-indeno[1,2-b]pyridin-5-on
   Die Mischung von 51 g (0,211 mol) 2-Phenyl-4-methylnicotinsäure-ethylester und 500 g Polyphosphorsäure wurden 7 Stunden bei 140°C gerührt, abgekühlt, in 700 g Eis eingerührt, mit konz. Ammoniaklösung alkalisch gestellt und mit Essigester extrahiert. Die vereinigten Extrakte wurden zweimal mit Wasser gewaschen, über K₂CO₃ getrocknet und eingeengt. Das so erhaltene Produkt wurde anschließend 2 Stunden mit Diethylether digeriert, abgesaugt und getrocknet. Ausbeute 18,6 g (45 % d. Theorie), gelbliche Kristalle vom Schmp. 137 - 139°C.
b) 2-Phenyl-4-methylnicotinsäure-ethylester
   Einer Suspension von 2 g Natriumhydrid in Dioxan wurden nacheinander bei 25°C und unter Stickstoffeinleitung 192 g (1 mol) Benzoylessigsäureethylester in 20 Min. und die Lösung aus 84 g (1,2 mol) Crotonaldehyd in 100 ml Dioxan in 30 Min. unter Rühren zugetropft. Nach 10stündigem Rühren wurde das Reaktionsgemisch mit 800 ml Essigsäure und anschließend mit 245 g (3,5 mol) Hydroxylaminchlorhydrat versetzt. Nach 2stündigem Rühren bei 110°C, wurde auf 20°C abgekühlt, das Gemisch in 1 kg Eis eingerührt und mit festem K₂CO₃ alkalisch gestellt. Anschließend wurde mit Essigester extrahiert, die vereinigten Extrakte mit 2N HCl-Lösung extrahiert und die vereinigten, wäßrigen Extrakte mit festem K₂CO₃ alkalisch gestellt und erneut mit dreimal je 300 ml Essigester extrahiert. Nach Einengen der vereinigten organischen Extrakte wurden 62 g 2-Phenyl-4-methylnicotinsäure-ethylester (88,5 % d. Theorie) als gelbliches Öl, n_{D}²³ = 1,5724, erhalten.

### Beispiel 2

### 4-Methyl-5H-indeno[1,2-b]pyridin-5-ol

Eine Lösung von 15 g (0,0769 mol) 4-Methyl-5H-indeno[1,2-b]pyridin-5-on in 150 ml Methanol wurde bei 0 bis -5°C mit 3,8 g (0,1 mol) NaBH₄ portionsweise versetzt. Nach 14stündigem Rühren bei Raumtemperatur wurde das Gemisch bei vermindertem Druck eingeengt. Der Rückstand wurde mit 350 ml Methylenchlorid und mit 150 ml einer 15 %igen, wäßrigen Kaliumhydroxydlösung versetzt und 1 Stunde nachgerührt. Die organische Phase wurde abgetrennt, mit Wasser gewaschen, über Na₂SO₄ getrocknet und im Vakuum bis zu einem Volumen von 50 ml eingeengt. Der Kristallbrei wurde bei 0°C abgesaugt und mit wenig Diethylether und Pentan gewaschen. Man erhielt 9,4 g (62 % d. Theorie) 4-Methyl-5H-indeno[1,2-b]pyridin-5-ol als gelbliche Kristalle vom Schmp. 154 - 156°C.

### Beispiel 3

### 4-Methyl-5H-indeno[1,2-b]pyridin-5-on-N-oxid

Einer Lösung von 13 g (0,066 mol) wasserfeuchter 3-Chlorperbenzoesäure in 500 ml Chloroform wurde bei 25°C die Lösung von 12,7 g (0,065 mol) 4-Methyl-5H-indeno[1,2-b]pyridin-5-on in 550 ml Chloroform zugetropft. Nach 12stündigem Rühren bei Raumtemperatur wurde das Gemisch zweimal mit je 50 ml einer gesättigten, wäßrigen Natriumhydrogencarbonatlösung, dann mit Wasser gewaschen, über Na₂SO₄ getrocknet und bei vermindertem Druck eingeengt. Der Rückstand wurde mit 20 ml Diethylether bei 0°C zur Kristallisation gebracht. Man erhielt 12,4g (90,5 % d. Theorie) 4-Methyl-5H-indeno[1,2-b]pyridin-5-on-N-oxid als weiße Kristalle von Schmp. 218 -220°C.

### Beispiel 4

### 4-Methyl-7-nitro-5H-indeno[1,2-b]pyridin-5-on

Einer Lösung von 15,6 g (0,08 mol) 4-Methyl-5H-indeno[1,2-b]pyridin-5-on in 35,2 ml konz. Schwefelsäure wurde bei + 5 bis 10°C die Mischung aus 35,2 ml 98,9 %iger Schwefelsäure mit 35,2 ml 99%iger Salpetersäure in 1 Stunde zugetropft. Nach 14-stündigem Rühren bei 25°C und 4-stündigem Nachrühren bei 70°C wurde das Gemisch auf 20°C abgekühlt, in 500 g Eis eingerührt und mit einer 50 %igen, wäßrigen Natriumhydroxydlösung auf pH 10 gebracht. Das Gemisch wurde mit Essigester extrahiert, die vereinigten Extrakte im Vakuum eingeengt und der Rückstand mit 30 ml Ether bei + 10°C kristallisiert. Man erhielt 13 g (67 %d. Theorie) 4-Methyl-7-nitro-5H-indeno[1,2-b]pyridin-5-on als gelbe Kristalle vom Schmp. 225 - 227°C.

### Beispiel 5

### 2-Acetyl-4-methyl-5H-indeno[1,2-b]pyridin-5-on

Einer Lösung von 19,5 g (0,1 mol) 4-Methyl-5H-indeno[1,2-b]pyridin-5-on in 500 ml Methylenchlorid werden nacheinander 500 ml Wasser, 20 g (0,2 mol) konz. Schwefelsäure, 26,4 g (0,3 mol) Brenztraubensäure, 1,7 g (0,01 mol) Silbernitrat und 34,2 g (0,15 mol) Ammoniumperoxodisulfat bei 25°C zugegeben. Nach 4-stündigem Rühren bei 40°C wurde das Gemisch auf +10°C abgekühlt, mit festem Kaliumcarbonat alkalisch gestellt, die organische Phase abgetrennt, mit Wasser gewaschen und unter vermindertem Druck eingeengt. Man erhielt 7,7 g (32, 4 % d. Theorie) 2-Acetyl-4-methyl-5H-indeno[1,2-b]pyridin-5-on als hellgelbe Kristalle von Schmp. 170 - 172°C.

### Beispiel 6

### 2,4-Dimethyl-5H-indeno[1,2-b]pyridin-5-on

Die Lösung von 23,6 g (0,24 mol) 4-Amino-3-penten-2-on, 29,2 g (0,2 mol) Inden-1,3-(2H)dion und 7,7 g (0,1 mol) Ammoniumacetat in 500 ml Essigsäure wurde 3 Stunden bei 115°C gerührt, unter vermindertem Druck eingeengt und der Rückstand mit 500 ml Wasser versetzt und mit festem Kaliumcarbonat alkalisch gestellt. Das Gemsich wurde dreimal mit Methylenchlorid extrahiert, die vereinigten Extrakte mit 100 ml Wasser gewaschen und im Vakuum eingeengt. Der Rückstand wurde mit 20 ml Diethylether bei + 5°C zur Kristallisation gebracht. Man erhielt 10,2 g 2,4-Dimethyl-5H-indeno[1,2-b]pyridin-5-on als schwach gelbe Kristalle vom Schmp. 102 -104°C.

### Beispiel 7

### 2-Ethyl-4-methyl-5H-indeno[1,2-b]pyridin-5-on

Einer Lösung von 11,7 g (0,006 mol) 4-Methyl-5H-indeno[1,2-b]pyridin-5-on in 130 ml 2N Schwefelsäure wurden bei 25°C 3,6 g Silbernitrat (0,018 mol) und 26,4 g (0,3 mol) Propionsäure und bei 70°C in 40 Minuten die Lösung von 27,4 g (0,12 mol) Ammoniumperoxodisulfat in 60 ml Wasser zugetropft. Das Gemisch wurde noch 90 Minuten bei 80°C gerührt, dann abgekühlt und bei ca. 10°C mit einer 28 %igen, wäßrigen Ammoniaklösung alkalisch gestellt, dreimal mit Methylenchlorid extrahiert und die vereinigten organischen Extrakte mit 50 ml Wasser gewaschen und eingeengt. Der Rückstand wird mit 5 ml Diethylether bei 0°C zur Kristallisation gebracht. Man erhielt 5,6 g (41,8 % d. Theorie) 2-Ethyl-4-methyl-5H-indeno[1,2-b]pyridin-5-on als schwach gelbe Kristalle von Schmp. 68 - 70°C.

### Beispiel 8

### 4-Methyl-5H-indeno[1,2-b]pyridin-5-O-methyloxim

Einer Lösung von 13 g (0,066 mol) 4-methyl-5H-indeno[1,2-b]pyridin-5-on und 12 g (0,14 mol) O-Methylhydroxylamin-chlorhydrat in 150 ml Methanol wurden 11,1 g (0,14 mol) Pyridin zugetropft. Das Gemisch wurde bei Raumtemperatur 12 Stunden und bei 50°C weitere 5 Stunden gerührt, abgekühlt und unter vermindertem Druck eingeengt. Der Rückstand wurde in 300 ml Methylenchlorid aufgelöst, dreimal mit Wasser gewaschen, über Na₂SO₄ getrocknet und im Vakuum eingeengt. Ma erhielt 6,3 g (42,6 % d. Theorie) 4-Methyl-5H-indeno[1,2-b]pyridin-5-O-methyloxim als weiße Kristalle vom Schmp. 94 -95°C.

### Beispiel 9

a) 1,3-Dimethyl-4-carboxymethyl-9H-indeno[2,1-c]pyridin-9-on
   50 g (0,167 mol) 2,6-Dimethyl-4-phenyl-3,5-bis(carboxymethyl)pyridin und 395 g Polyphosphorsäure wurden 3 Tage bei 170°C gerührt, auf 50°C abgekühlt, mit 800 g Eis verdünnt und mit einer 28 %igen, °ßrigen Ammoniaklösung alkalisch gestellt. Das Gemisch wurde zweimal mit Methylenchlorid extrahiert und die vereinigten Extrakte im Vakuum eingeengt. Man erhielt die Titelverbindung als gelbe Kristalle vom Schmp. 152 -154°C. Ausbeute 7,8 g (17,4 % d. Theorie).
b) Herstellung von 2,6-Dimethyl-4-phenyl-3,5-bis(carboxymethyl)pyridin
   Einer Lösung von 375 g 98 %iger Salpetersäure in 1580 ml Wasser wurden bei 92 bis 98°C portionsweise 90,3 g (0,3 mol) 1,4-Dihydro-2,6-dimethyl-4-phenyl-3,5-bis(carboxymethyl)pyridin in 30 Minuten zugegeben. Nach weiteren 30 Minuten bei 95 - 98°C wurde das Gemisch auf +5°C abgekühlt und mit festem Kaliumcarbonat auf pH 9 gebracht. Das Gemisch wurde dreimal mit Methylenchlorid extrahiert, die vereinigten Extrakte unter vermindertem Druck eingeengt und der Rückstand mit 1:1 Diethylether-Pentan (50 ml) bei 0°C zur Kristallisation gebracht. Man erhielt 58,8 g (65,5 % d. Theorie) 2,6-Dimethyl-4-phenyl-3,5-bis(carboxymethyl)pyridin als weiße Kristalle vom Schmp. 137 - 138°C.

Analog zu den Herstellungsbeispielen 1 bis 9 und gemäß den allgemeinen Angaben zur Herstellung erhielt man die in Tabelle 2 aufgeführten Verbindungen der allgemeinen Formel I/Ia:

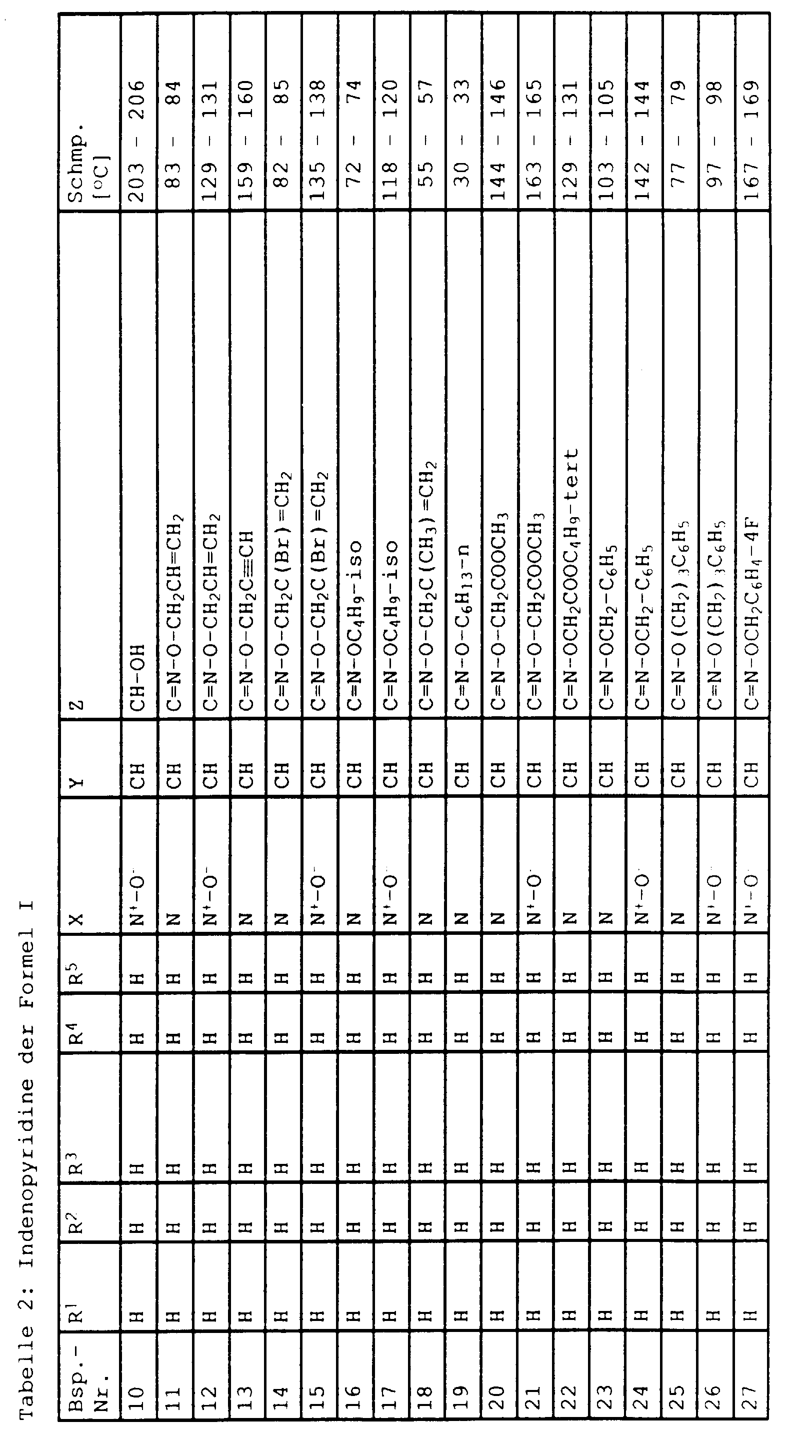

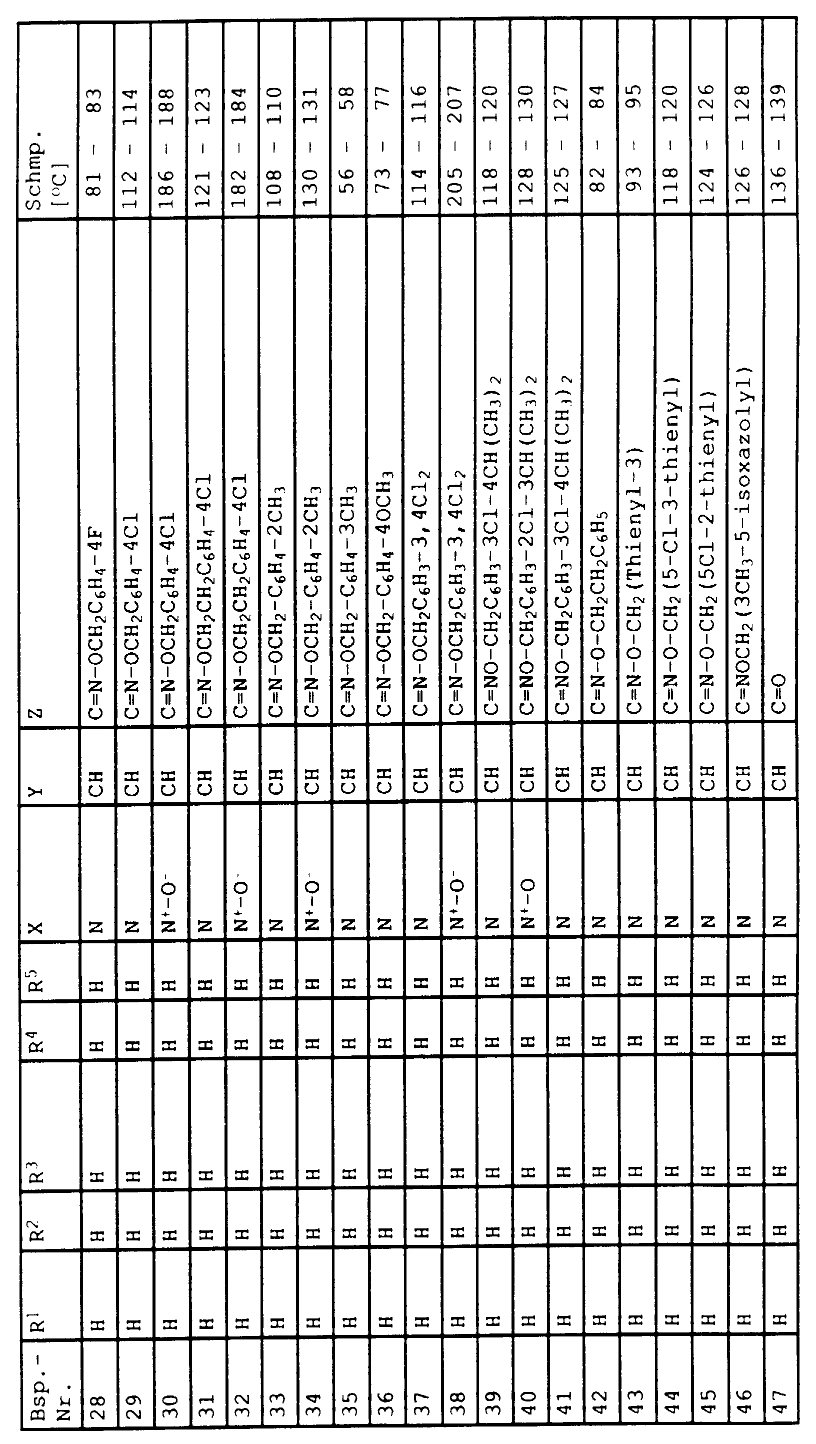

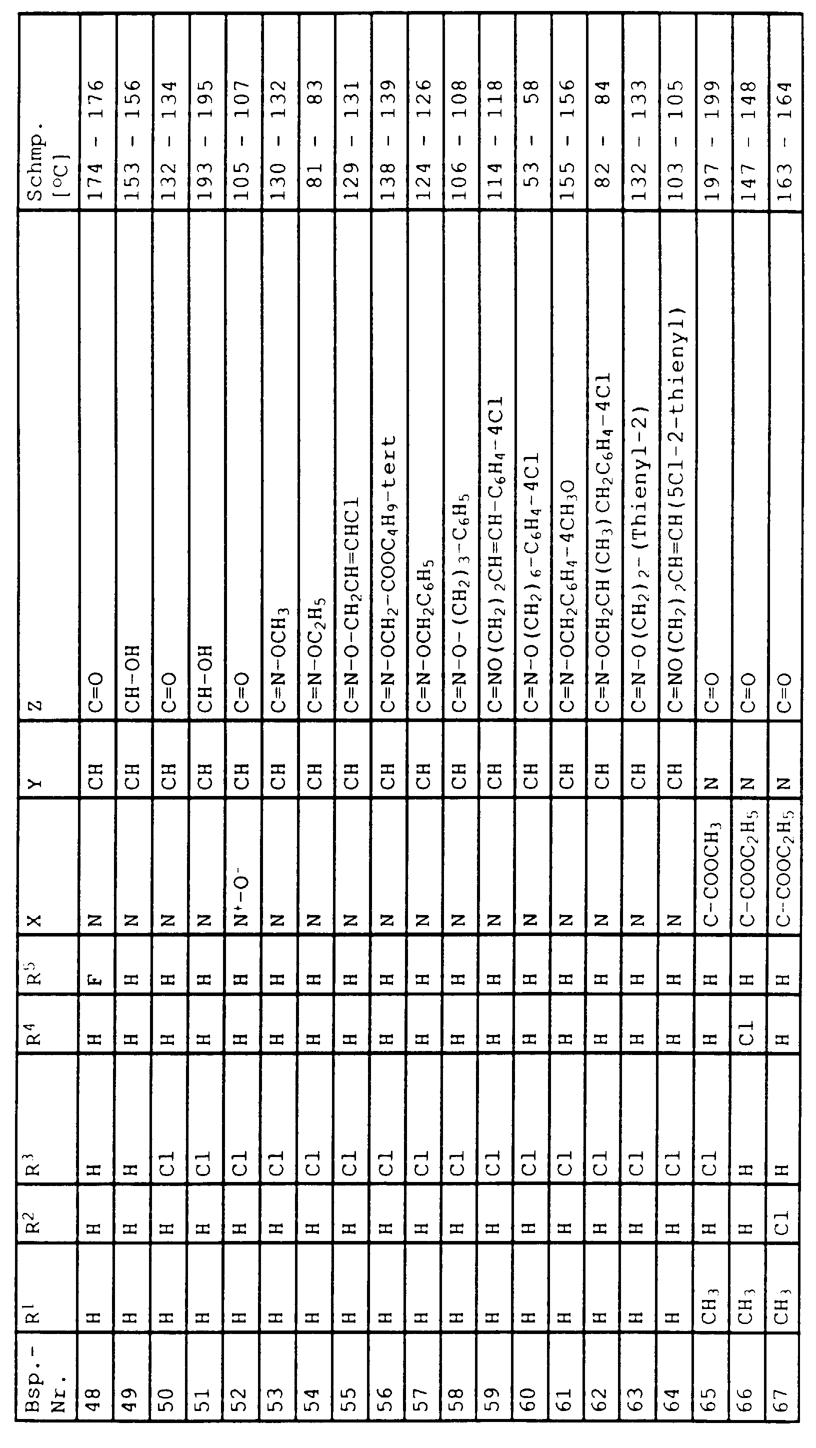

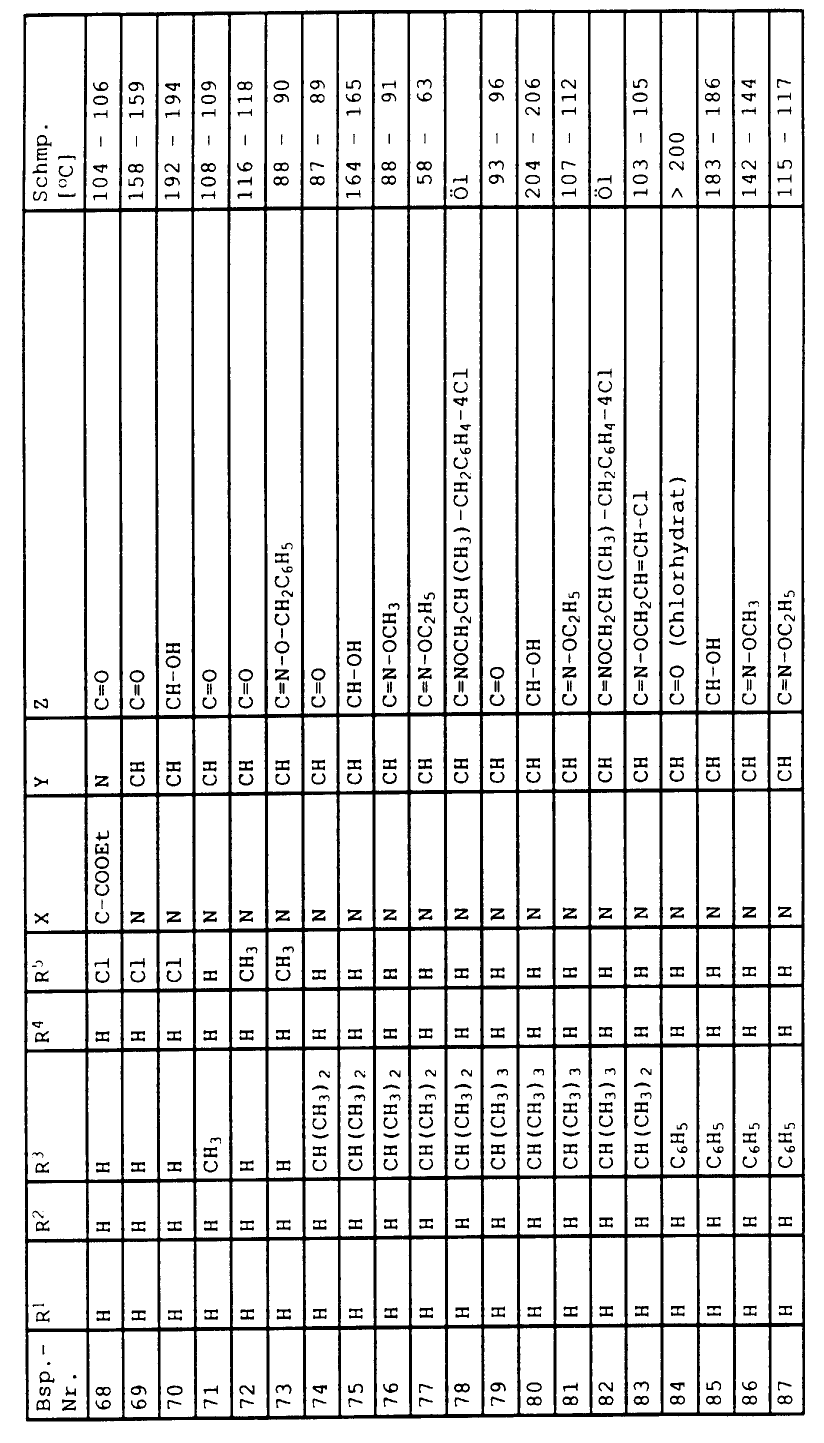

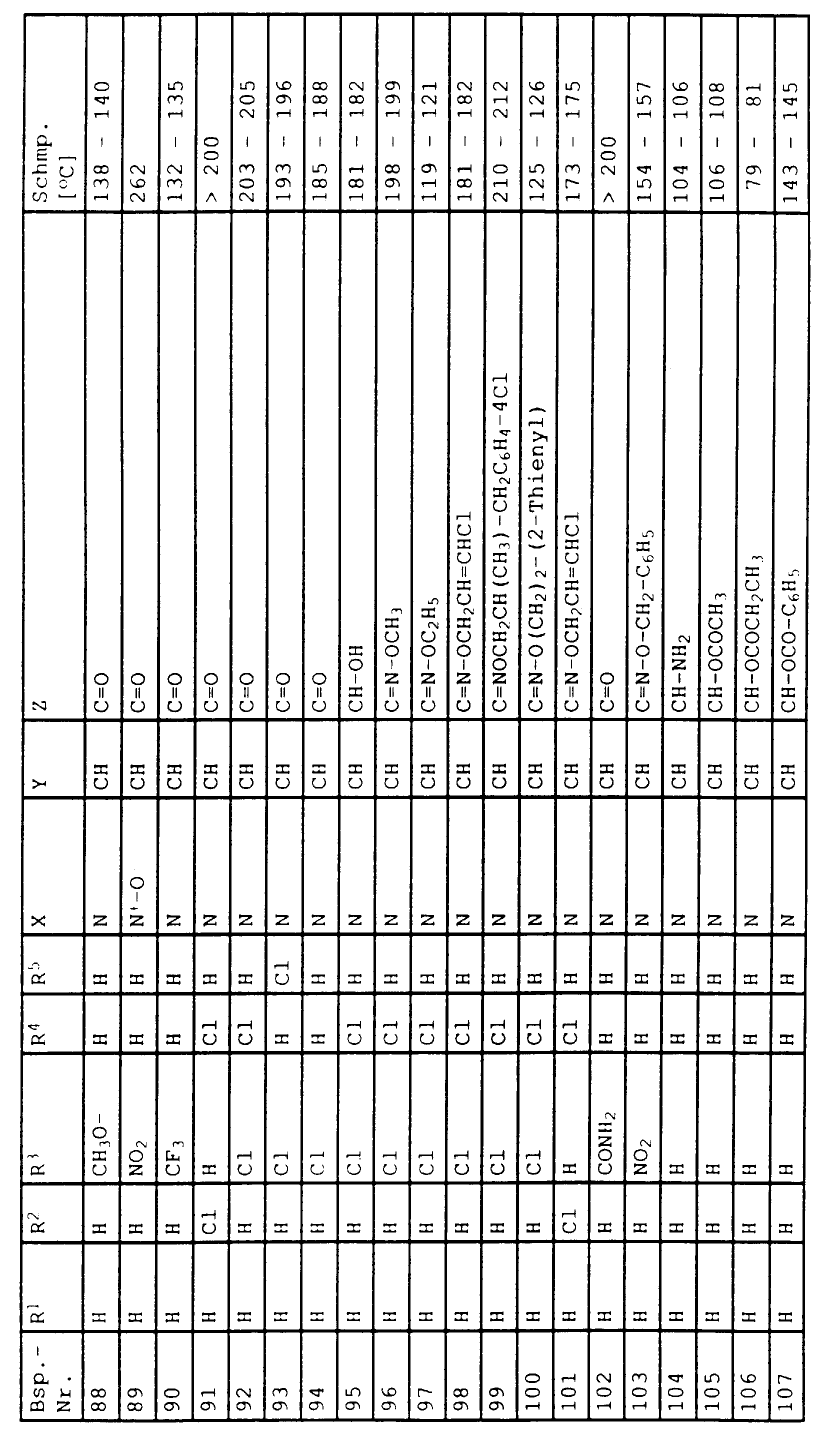

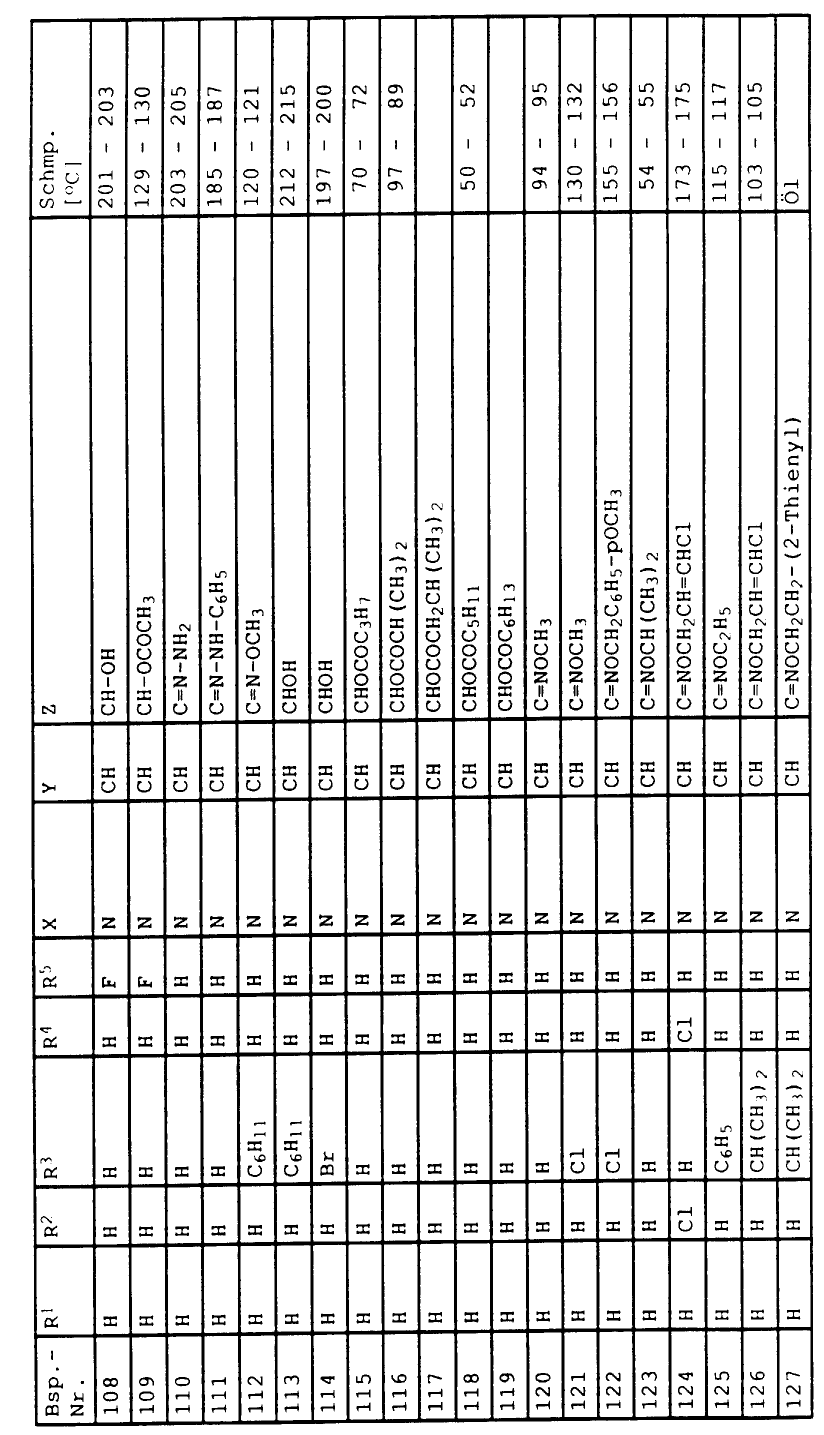

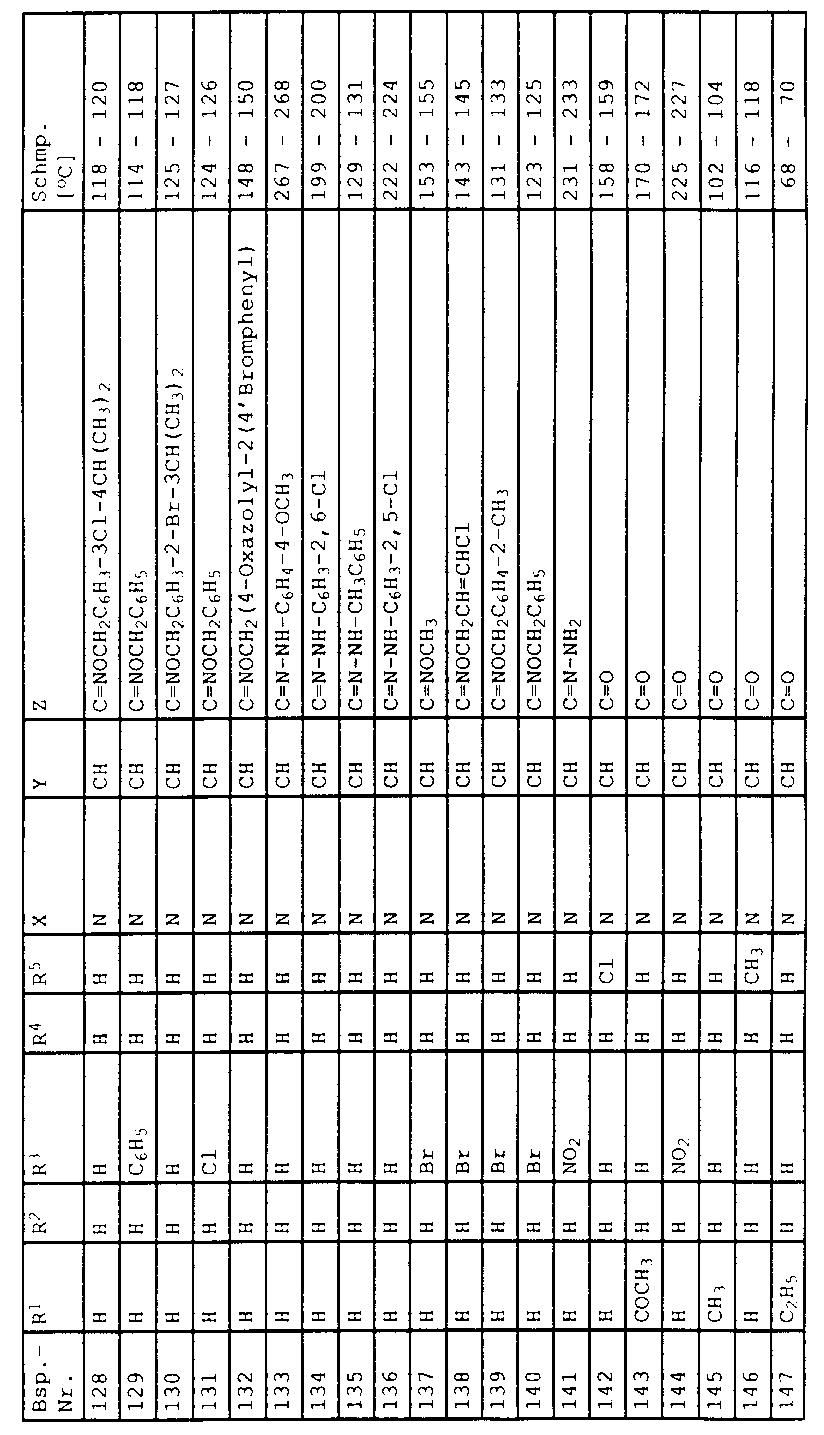

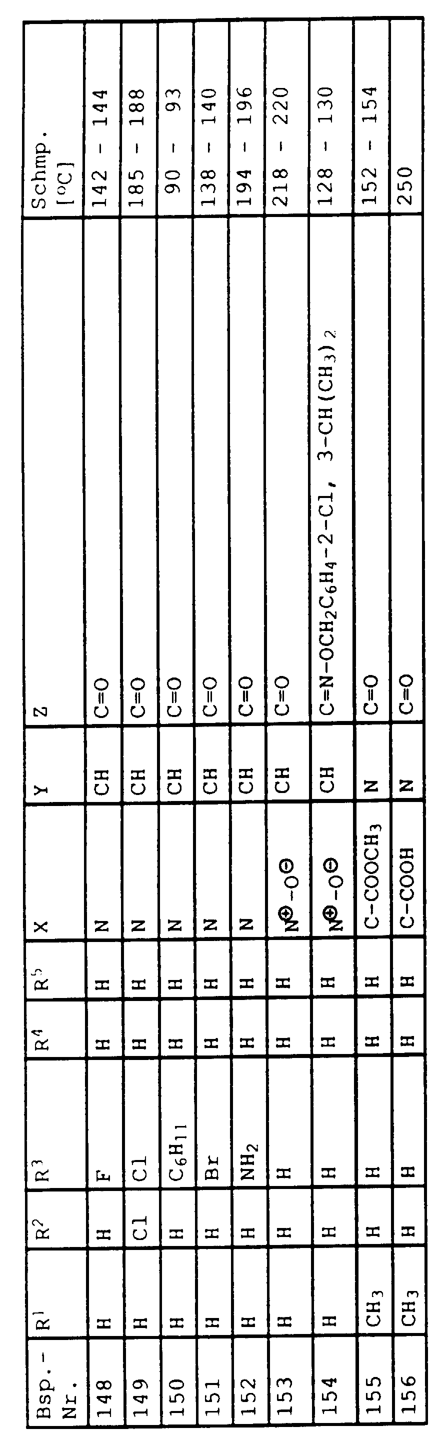

### Anwendungsbeispiele

Die herbizide Wirkung der Indenopyridine der Formel I bzw. I' ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 0,5 bis 3,0 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Bromus spp. | Trespe | brome |
| Centaurea cyanus | Kornblume | cornflower |
| Echinochloa crus-galli | Hühnerhirse | barnyardgrass |
| Setaria italica | Ital. Borstenhirse | millet foxtail |
| Digitaria sanguinalis | Blutfingerhirse | large crabgrass |
| Brassica napus | Winterraps | oilseed rape |

Die Ergebnisse (s. Tabellen I-III) belegen die überlegene herbizide Wirkung der erfindungsgemäßen Verbindung Nr. 48 im Vergleich zu folgenden aus dem Stand der Technik bekannten Vergleichsverbindungen:

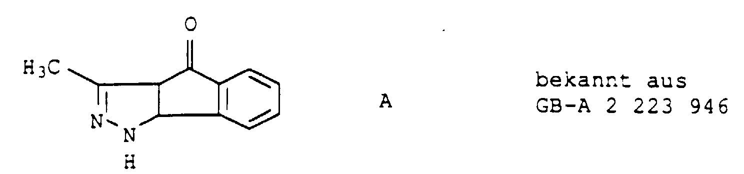

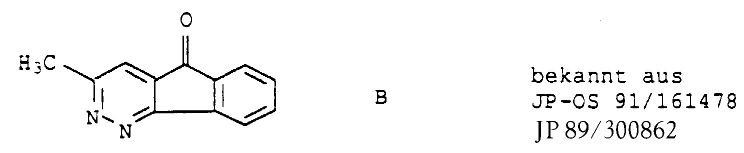

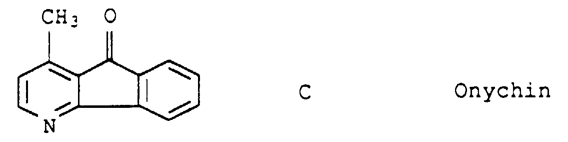

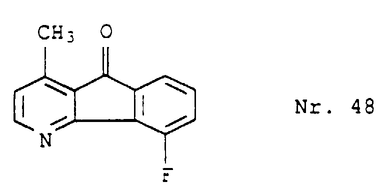

Verbindung Nr. 11 zeigte in der Beispielkultur Winterraps eine sehr gute wachstumshemmende Wirkung bei einer Aufwandmenge von 0,5 kg/ha. Verbindung Nr. 75 zeigt bei einer Aufwandmenge von 0,5 kg/ha a.S. eine gute herbizide Wirkung bei gleichzeitig hoher Selektivität in den Kulturen Sommerweizen und Baumwolle.

**Tabelle I**

| Beispiele zur Bekämpfung unerwünschter Pflanzen bei Nachauflaufanwendung von 3,0 kg a.S./ha im Gewächshaus | | |
|---|---|---|
| | Schädigung in % | |
| Testpflanzen | Bsp.-Nr. 48 | A |
| Bromus spp. | 100 | 15 |
| Centaurea cyanus | 100 | 0 |
| Echinochloa crus-galli | 100 | 40 |

**Tabelle II**

| Beispiele zur Bekämpfung unerwünschter Pflanzen bei Nachauflaufanwendung im Gewächshaus | | |
|---|---|---|
| | Schädigung in % | |
| Bsp.-Nr. | 48 | B |
| Aufwandmenge (kg a.S./ha) | 1,0 | 1,5 |
| Testpflanzen | | |
| Centaurea cyanus | 95 | 20 |
| Echinochloa crus-galli | 84 | 0 |
| Setaria italica | 100 | 85 |

**Tabelle III**

| Beispiele zur Bekämpfung unerwünschter Pflanzen bei Nachauflaufanwendung im Gewächshaus | | | | |
|---|---|---|---|---|
| | Schädigung in % | | | |
| Bsp.-Nr. | 48 | 48 | C | C |
| Aufwandmenge (kg a.S./ha) | 1,0 | 0,5 | 1,0 | 0,5 |
| Testpflanzen | | | | |
| Centaurea cyanus | 95 | 50 | 50 | 20 |
| Digitaria sanguinalis | 100 | 100 | 90 | 60 |
| Echinochloa crus-galli | 90 | 60 | 40 | 40 |

## Patentansprüche

1. 4-Methyl-5H-indeno[1,2-b]pyridine und 1-Methyl-9H-indeno[2,1-c]pyridine der allgemeinen Formel I 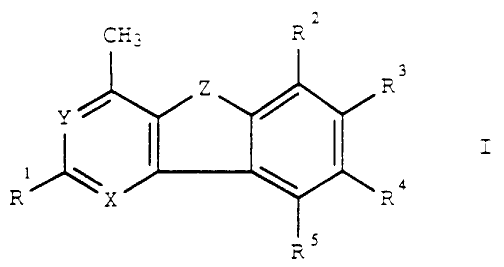 in der X, Y, Z und die Reste R¹ bis R⁵ folgende Bedeutung haben:
R¹ Wasserstoff, C₁-C₆-Alkyl, eine Gruppe COR⁶, wobei R⁶ für C₁-C₄-Alkyl steht,
R²-R⁵
a) Wasserstoff,
b) Halogen,
c) Nitro,
d) COOR⁶, wobei R⁶ für C₁-C₄-Alkyl steht,
e) CONH₂ und CONR⁶R⁷, wobei R⁶ und R⁷ C₁-C₄-Alkyl bedeuten,
f) C₁-C₈-Alkyl, das ein bis drei der folgenden Substituenten tragen kann: Halogen, Hydroxy, C₁-C₆-Alkoxy,
g) C₃-C₆-Alkenyl,
h) C₁-C₄-Alkoxy,
i) Hydroxy,
j) Amino oder NR⁶R⁷,
k) Phenyl, das ein bis fünf Halogenatome oder ein bis drei der folgenden Substituenten tragen kann: Nitro, C₁-C₄-Alkoxy, C₁-C₄-Alkyl oder C₁-C₄-Halogenalkyl;
X N, N⁺-O⁻;
Y CR⁸, wobei R⁸ für Wasserstoff, C₁-C₂-Alkyl, COOH oder COOR⁶ steht;
Z eine Gruppe C=O, CH-OR⁹ CH-O-COR⁹ oder C=N-W-R⁹, wobei W für Sauerstoff oder die Gruppierung - N(R¹⁰) steht und in der
R⁹ Wasserstoff, eine C₁-C₆-Alkylgruppe, die durch Halogen, COOR⁶ oder C₁-C₄-Alkoxy substituiert sein kann, eine C₃-C₆-Alkenylgruppe, eine C₃-C₆-Halogenalkenylgruppe, eine C₃-C₆-Alkinylgruppe, ferner Phenyl-, Phenyl-C₁-C₄-alkyl oder einkernige Heteroaryl- oder Heteroaryl-C₁-C₄-alkylreste bedeutet, wobei Heteroaryl jeweils für einen heteroaromatischen Rest mit 5 bis 6 Ringgliedern, enthaltend 1 bis 3 Heteroatome wie N, S oder O steht und wobei diese aromatischen bzw. heteroaromatischen Gruppen ein bis drei der folgenden Substituenten am Kern tragen können: Halogen, Nitro, C₁-C₆-Alkyl, C₁-C₆-Alkoxy, C₁-C₂-Halogenalkyl, Amino, C₁-C₆-Alkylamino, Di(C₁-C₆-alkyl)amino und
R¹⁰ Wasserstoff oder eine C₁-C₄-Alkylgruppe bedeutet,
sowie die Salze von I mit solchen Säuren, welche die herbizide bzw. die pflanzenwachstumsregulierende Wirkung von I nicht beeinträchtigen, ausgenommen Onychin, 6-, 7-, 8- und 9-Methoxyonychin, 6-, 7-, 8- und 9-Hydroxyonychin, 7,8-Dimethoxyonychin und 4-Methyl-5H-indeno[1,2-b]pyridine der Formel Ia 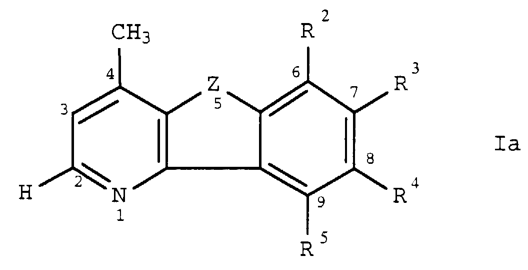 in der zwei der Reste R², R³, R⁴ oder R⁵ eine Kombination aus Methoxy und Hydroxy bilden, wenn Z für C=O oder CHOH steht und die verbleibenden zwei Reste Wasserstoff oder Methoxy bedeuten.

2. Herbizides Mittel, enthaltend eine herbizid wirksame Menge einer Verbindung der Formel I gemäß Anspruch 1.

3. Mittel zur Regulierung des Pflanzenwachstums, enthaltend eine bioregulatorisch wirksame Menge einer Verbindung der Formel I, gemäß Anspruch 1 und inerte Zusatzstoffe.

4. Verfahren zur Bekämpfung unerwünschten Pflanzenwuchses, dadurch gekennzeichnet, daß man eine herbizid wirksame Menge einer Verbindung der Formel I' 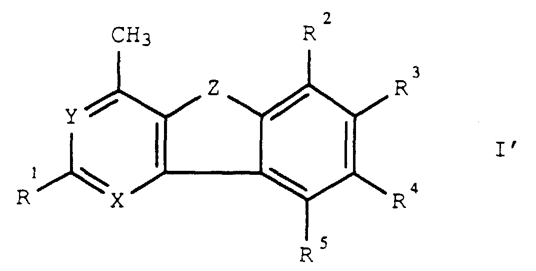 in der X, Y, Z und die Reste R¹ bis R⁵ folgende Bedeutung haben:
R¹ Wasserstoff, C₁-C₆-Alkyl, eine Gruppe COR⁶, wobei R⁶ für C₁-C₄-Alkyl steht,
R²-R⁵
a) Wasserstoff,
b) Halogen,
c) Nitro,
d) COOR⁶, wobei R⁶ für C₁-C₄-Alkyl steht,
e) CONH₂ und CONR⁶R⁷, wobei R⁶ und R⁷ C₁-C₄-Alkyl bedeuten,
f) C₁-C₈-Alkyl, das ein bis drei der folgenden Substituenten tragen kann: Halogen, Hydroxy, C₁-C₆-Alkoxy,
g) C₃-C₆-Alkenyl,
h) C₁-C₄-Alkoxy,
i) Hydroxy,
j) Amino oder NR⁶R⁷,
k) Phenyl, das ein bis fünf Halogenatome oder ein bis drei der folgenden Substituenten tragen kann: Nitro, C₁-C₄-Alkoxy, C₁-C₄-Alkyl oder C₁-C₄-Halogenalkyl;
X, Y N, N⁺-O⁻ oder CR⁸, wobei R⁸ für Wasserstoff, C₁-C₂-Alkyl, COOH oder COOR⁶ steht, mit der Maßgabe, daß ausschließlich ein Stickstoffatom bzw. eine N-Oxidgruppe im Ring enthalten ist;
Z eine Gruppe C=O, CH-OR⁹ CH-O-COR⁹ oder C=N-W-R⁹, wobei W für Sauerstoff oder die Gruppierung - N(R¹⁰) steht und in der
R⁹ Wasserstoff, eine C₁-C₆-Alkylgruppe, die durch Halogen, COOR⁶ oder C₁-C₄-Alkoxy substituiert sein kann, eine C₃-C₆-Alkenylgruppe, eine C₃-C₆-Halogenalkenylgruppe, eine C₃-C₆-Alkinylgruppe, ferner Phenyl-, Phenyl-C₁-C₄-alkyl oder einkernige Heteroaryl- oder Heteroaryl-C₁-C₄-alkylreste bedeutet, wobei diese aromatischen bzw. heteroaromatischen Gruppen ein bis drei der folgenden Substituenten am Kern tragen können: Halogen, Nitro, C₁-C₆-Alkyl, C₁-C₆-Alkoxy, C₁-C₂-Halogenalkyl, Amino, C₁-C₆-Alkylamino, Di(C₁-C₆-alkyl)amino und
R¹⁰ Wasserstoff oder eine C₁-C₄-Alkylgruppe bedeutet,
sowie die Salze von I mit solchen Säuren, welche die herbizide bzw. die pflanzenwachstumsregulierende Wirkung von I nicht beeinträchtigen, auf Pflanzen, deren Lebensraum oder auf Saatgut einwirken läßt.

5. Verwendung von Verbindungen der Formel I' gemäß Anspruch 4 zur Herstellung herbizid oder bioregulatorisch wirksamer Mittel.

## Claims

1. A 4-methyl-5H-indeno[3,2-b]pyridine or 1-methyl-9H-indeno[2,3-c]pyridine of the general formula I 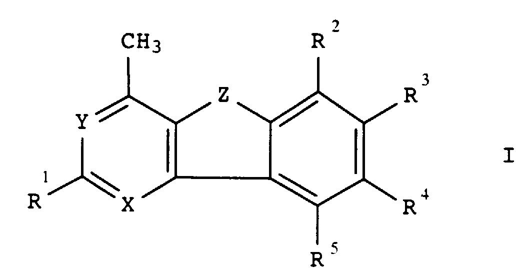 where X, Y, Z and the radicals R¹ to R⁵ have the following meanings:
R¹ is hydrogen, C₁-C₆-alkyl or a COR⁶ group, R⁶ being C₁-C₄-alkyl,
R²-R⁵ are
a) hydrogen,
b) halogen,
c) nitro,
d) COOR⁶, R⁶ being C₁-C₄-alkyl,
e) CONH₂ or CONR⁶R⁷, R⁶ and R⁷ being C₁-C₄-alkyl,
f) C₁-C₈-alkyl which can carry one to three of the following substituents: halogen, hydroxyl or C₁-C₆-alkoxy,
g) C₃-C₆-alkenyl,
h) C₁-C₄-alkoxy,
i) hydroxyl,
j) amino or NR⁶R⁷,
k) phenyl which can carry one to five halogen atoms or one to three of the following substituents: nitro, C₁-C₄-alkoxy, C₁-C₄-alkyl or C₁-C₄-haloalkyl;
X is N, N⁺-O⁻;
Y is CR⁸, R⁸ being hydrogen, C₁-C₂-alkyl, COOH or COOR⁶;
Z is a C=O, CH-OR⁹, CH-O-COR⁹ or C=N-W-R⁹ group, W being oxygen or the -N(R¹⁰) group and where
R⁹ is hydrogen, a C₁-C₆-alkyl group which can be substituted by halogen, COOR⁶ or C₁-C₄-alkoxy, a C₃-C₆-alkenyl group, a C₃-C₆-haloalkenyl group, a C₃-C₆-alkynyl group, or phenyl, phenyl-C₁-C₄-alkyl or mononuclear heteroaryl or heteroaryl-C₁-C₄-alkyl radicals, heteroaryl in each case being a heteroaromatic radical having 5 to 6 ring members, containing 1 to 3 heteroatoms such as N, S or O and these aromatic or heteroaromatic groups being able to carry one to three of the following substituents on the ring: halogen, nitro, C₁-C₆-alkyl, C₁-C₆-alkoxy, C₁-C₂-haloalkyl, amino, C₁-C₆-alkylamino or di(C₁-C₆-alkyl)amino and
R¹⁰ is hydrogen or a C₁-C₄-alkyl group,
and the salts of I with those acids which do not adversely affect the herbicidal or the plant growth-regulating action of I, excluding onychine, 6-, 7-, 8- and 9-methoxyonychine, 6-, 7-, 8- and 9-hydroxyonychine, 7,8-dimethoxyonychine and 4-methyl-5H-indeno-[3,2-b]pyridines of the formula Ia 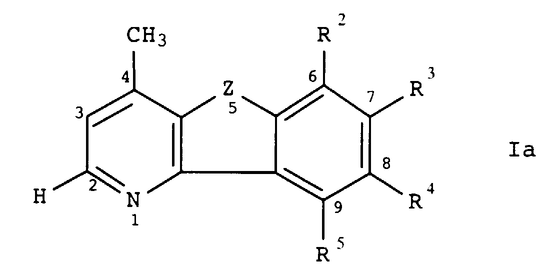 where two of the radicals R², R³, R⁴ and R⁵ form a combination of methoxy and hydroxyl if Z is C=O or CHOH and the remaining two radicals are hydrogen or methoxy.

2. A herbicidal composition, containing a herbicidally effective amount of a compound of the formula I as claimed in claim 1.

3. A composition for regulating plant growth, containing a bioregulatory amount of a compound of the formula I as claimed in claim 1, and inert additives.

4. A method for controlling undesired plant growth, which comprises allowing a herbicidally effective amount of a compound of the formula I' 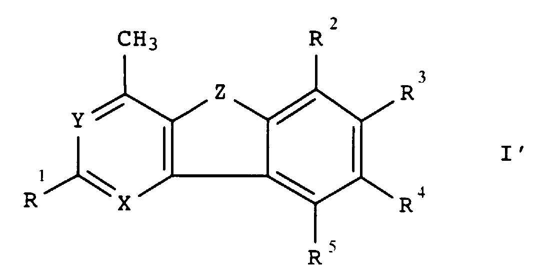 where X, Y, Z and the radicals R¹ to R⁵ have the following meanings:
R¹ is hydrogen, C₁-C₆-alkyl or a COR⁶ group, R⁶ being C₁-C₄-alkyl,
R²-R⁵ are
a) hydrogen,
b) halogen,
c) nitro,
d) COOR⁶, R⁶ being C₁-C₄-alkyl,
e) CONH₂ or CONR⁶R⁷, R⁶ and R⁷ being C₁-C₄-alkyl,
f) C₁-C₈-alkyl which can carry one to three of the following substituents: halogen, hydroxyl or C₁-C₆-alkoxy,
g) C₃-C₆-alkenyl,
h) C₁-C₄-alkoxy,
i) hydroxyl,
j) amino or NR⁶R⁷,
k) phenyl which can carry one to five halogen atoms or one to three of the following substituents: nitro, C₁-C₄-alkoxy, C₁-C₄-alkyl or C₁-C₄-haloalkyl;
X and Y are N, N⁺-O⁻ or CR⁸, R⁸ being hydrogen, C₁-C₂-alkyl, COOH or COOR⁶, with the proviso that exclusively one nitrogen atom or one N-oxide group is contained in the ring;
Z is a C=O, CH-OR⁹, CH-O-COR⁹ or C=N-W-R⁹ group, W being oxygen or the -N(R¹⁰) group and where
R⁹ is hydrogen, a C₁-C₆-alkyl group which can be substituted by halogen, COOR⁶ or C₁-C₄-alkoxy, a C₃-C₆-alkenyl group, a C₃-C₆-haloalkenyl group, a C₃-C₆-alkynyl group, or phenyl, phenyl-C₁-C₄-alkyl or mononuclear heteroaryl or heteroaryl-C₁-C₄-alkyl radicals, these aromatic or heteroaromatic groups being able to carry one to three of the following substituents on the ring: halogen, nitro, C₁-C₆-alkyl, C₁-C₆-alkoxy, C₁-C₂-haloalkyl, amino, C₁-C₆-alkylamino or di(C₁-C₆-alkyl)amino and
R¹⁰ is hydrogen or a C₁-C₄-alkyl group,
and the salts of I' with those acids which do not adversely affect the herbicidal or the plant growth-regulating action of I' to act on plants, their habitat or on seeds.

5. The use of compounds of the formula I' as set forth in claim 4 for the production of herbicidal or bioregulatory compositions.

## Revendications

1. 4-méthyl-5H-indéno[1,2-b]pyridines et 1-méthyl-9H-indéno[2,1-c]pyridines, de formule générale I 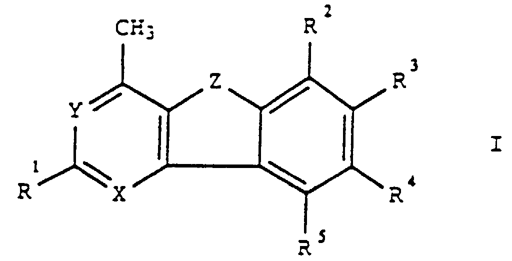 dans laquelle X, Y, Z et R¹ à R⁵ ont les significations suivantes :
R¹ : l'hydrogène, un groupe alkyle en C1-C6, un groupe COR⁶ dans lequel R⁶ représente un groupe alkyle en C1-C4,
R² à R⁵ :
a) l'hydrogène,
b) un halogène,
c) un groupe nitro,
d) un groupe COOR⁶ dans lequel R⁶ représente un groupe alkyle en C1-C4,
e) un groupe CONH₂ ou CONR⁶R⁷ dans lequel R⁶ et R⁷ représentent des groupes alkyle en C1-C4,
f) un groupe alkyle en C1-C8 qui peut porter un à trois des substituants suivants : halogéno, hydroxy, alcoxy en C1-C6,
g) un groupe alcényle en C3-C6,
h) un groupe alcoxy en C1-C4,
i) un groupe hydroxy,
j) un groupe amino ou NR⁶R⁷,
k) un groupe phényle, qui peut porter un à cinq atomes d'halogènes ou un à trois des substituants suivants : nitro, alcoxy en C1-C4, alkyle en C1-C4 ou halogénoalkyle en C1-C4 ;
X: N, N⁺-O⁻ ;
Y : un groupe CR⁸ dans lequel R⁸ représente l'hydrogène, un groupe alkyle en C1-C2, COOH ou COOR⁶ ;
Z : un groupe C=O, CH-OR⁹, CH-O-COR⁹ ou C=N-W-R⁹, W représentant l'oxygène ou le groupement -N(R¹⁰)- et dans lequel
R⁹ représente l'hydrogène, un groupe alkyle en C1-C6 qui peut être substitué par des halogènes, des groupes COOR⁶ ou alcoxy en C1-C4, un groupe alcényle en C3-C6, un groupe halogénoalcényle en C3-C6, un groupe alcynyle en C3-C6, ou encore un groupe phényle, phénylalkyle en C1-C4 ou un groupe hétéroaryle ou hétéroaryl-alkyle en C1-C4 monocyclique, la partie hétéroaryle en question consistant en un radical hétéroaromatique à cinq ou six chaînons contenant un à trois hétéroatomes tels que N, S ou O, et les groupes aromatiques et hétéroaromatiques en question pouvant porter sur le cycle un à trois des substituants suivants : halogéno, nitro, alkyle en C1-C6, alcoxy en C1-C6, halogénoalkyle en C1-C2, amino, alkylamino en C1-C6, di(alkyle en C1-C6)amino et
R¹⁰ représente l'hydrogène ou un groupe alkyle en C1-C4,
et les sels de I et des acides qui n'affectent pas leur activité herbicide et régulatrice de la croissance des végétaux, à l'exception de l'onychine, de la 6-, de la 7-, de la 8- et de la 9-méthoxyonyquine, de la 6-, de la 7-, de la 8- et de la 9-hydroxyonychine, de la 7,8-diméthoxyonychine et des 4-méthyl-5H-indéno[1,2-b]pyridines de formule Ia 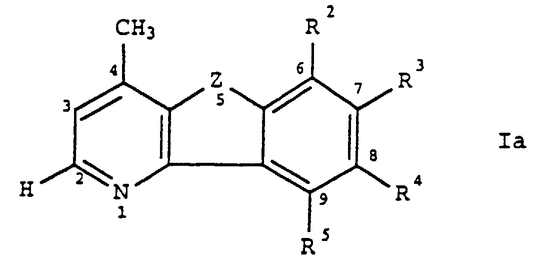 dans laquelle deux des symboles R², R³, R⁴ ou R⁵ forment une combinaison de groupes méthoxy et hydroxy, lorsque Z représente C=O ou CHOH, et les deux autres symboles représentent l'hydrogène ou des groupes méthoxy.

2. Produit herbicide contenant une quantité herbicide efficace d'un composé de formule I de la revendication 1.

3. Produit pour la régulation de la croissance des végétaux, contenant une quantité biorégulatrice efficace d'un composé de formule I selon la revendication 1, et des additifs inertes.

4. Procédé pour combattre les croissances des végétaux indésirables, caractérisé par le fait que l'on fait agir sur les végétaux, leur habitat ou sur les semences, une quantité herbicide efficace d'un composé de formule I' 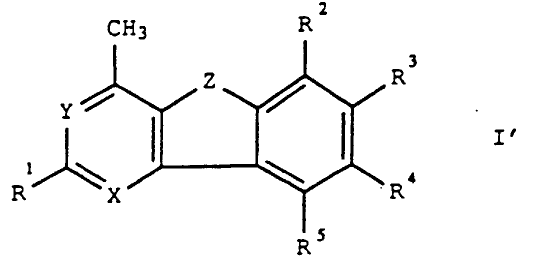 dans laquelle X, Y, Z et R¹ à R⁵ ont les significations suivantes :
R¹ : l'hydrogène, un groupe alkyle en C1-C6, un groupe COR⁶ dans lequel R⁶ représente un groupe alkyle en C1-C4,
R² à R⁵ :
a) l'hydrogène,
b) un halogène,
c) un groupe nitro,
d) un groupe COOR⁶ dans lequel R⁶ représente un groupe alkyle en C1-C4,
e) un groupe CONH₂ ou CONR⁶R⁷ dans lequel R⁶ et R⁷ représentent des groupes alkyle en C1-C4,
f) un groupe alkyle en C1-C8 qui peut porter un à trois des substituants suivants : halogéno, hydroxy, alcoxy en C1-C6,
g) un groupe alcényle en C3-C6,
h) un groupe alcoxy en C1-C4,
i) un groupe hydroxy,
j) un groupe amino ou NR⁶R⁷,
k) un groupe phényle qui peut porter un à cinq atomes d'halogènes ou un à trois des substituants suivants : nitro, alcoxy en C1-C4, alkyle en C1-C4 ou halogénoalkyle en C1-C4 ;
X, Y : N, N⁺-O⁻ ou CR⁸ dans lequel R⁸ représente l'hydrogène, un groupe alkyle en C1-C2, COOH ou COOR⁶, sous réserve que le cycle contient exclusivement un atome d'azote ou un groupe N-oxyde ;
Z : un groupe C=O, CH-OR⁹, CH-O-COR⁹ ou C=N-W-R⁹, W représentant l'oxygène ou le groupement N(R¹⁰), et dans lequel
R⁹ représente l'hydrogène, un groupe alkyle en C1-C6 qui peut être substitué par des halogènes, des groupes COOR⁶ ou alcoxy en C1-C4, un groupe alcényle en C3-C6, un groupe halogénoalcényle en C3-C6, un groupe alcynyle en C3-C6, ou encore un groupe phényle, phénylalkyle en C1-C4 ou un groupe hétéroaryle ou hétéroaryl-alkyle en C1-C4 monocyclique, ces groupes aromatiques et hétéroaromatiques pouvant porter sur le noyau un à trois des substituants suivants : halogéno, nitro, alkyle en C1-C6, alcoxy en C1-C6, halogénoalkyle en C1-C2, amino, alkylamino en C1-C6, di-(alkyle en C1-C6)amino et
R¹⁰ représente l'hydrogène ou un groupe alkyle en C1-C4,
ou encore les sels de I et d'acides qui n'affectent pas l'activité herbicide et régulatrice de croissance des composés I.

5. Utilisation des composés de formule I' selon la revendication 4, pour la préparation de produits à activité herbicide ou biorégulatrice.
